# EUROPEAN PATENT SPECIFICATION

(11) **EP 4 188 410 B1**
(45) Date of publication and mention of the grant of the patent: **05.11.2025**
(21) Application number: 21849618.0
(22) Date of filing: 30.07.2021
(51) Int. Cl.: A61K 38/17, A61K 38/30, C12N 15/62, A61P 25/28, A61K 38/00

(54) **CHIMERIC PROTEINS FOR USE IN THE TREATMENT OF CENTRAL NERVOUS SYSTEM DISORDERS**
CHIMÄRE PROTEINE ZUR VERWENDUNG BEI DER BEHANDLUNG VON ERKRANKUNGEN DES ZENTRALEN NERVENSYSTEMS
PROTÉINES CHIMÉRIQUES POUR L'UTILISATION DANS LE TRAITEMENT DE TROUBLES DU SYSTÈME NERVEUX CENTRAL

(30) Priority: 30.07.2020 US 202063058888 P
(43) Date of publication of application: 07.06.2023
(73) Proprietor: Silver Creek Pharmaceuticals, Inc., San Francisco, California 94158 (US)
(72) Inventor: PFAFF, Samuel J., San Francisco, CA 94132 (US); ZHANG, Yan, San Francisco, CA 94121 (US); KUCHENBECKER, Kristopher M., Phoenix, AZ 85018 (US); CHAWLA, Lakhmir S., San Diego, CA 92130 (US)
(74) Representative: Mewburn Ellis LLP
(86) International application number: PCT/US2021/044019
(87) International publication number: WO 2022/026902

(56) References cited:
- WO-A1-2011/146902
- WO-A2-2008/079290
- WO-A2-2012/145183
- CN-A- 105 524 176
- US-A1- 2020 207 826

## Description

### TECHNICAL FIELD

Aspects of the present disclosure relates generally to chimeric proteins and pharmaceutical compositions comprising such chimeric proteins, and methods for using such chimeric proteins for treating central nervous system disorders in a subject in need thereof.

### BACKGROUND

Brain injuries and central nervous system disorders are common medical condition that can result in disabling and irreversible degradation of a person's cognitive and sensorimotor capacity. Brain injuries and central nervous system disorders that can result in nerve cell death and damage range from ischemic stroke to degenerative disorders.

WO 2011/146902 discloses bi-specific fusion proteins with therapeutic uses, as well as pharmaceutical compositions comprising such fusion proteins, and methods for using such fusion proteins to repair or regenerate damaged or diseased tissue. CN 105524176 discloses bi-specific fusion proteins with therapeutic uses, as well as pharmaceutical compositions comprising such fusion proteins, and methods for using such fusion proteins to repair damaged tissue. US 2020/0207826 discloses bi-specific fusion proteins with therapeutic uses, as well as pharmaceutical compositions comprising such fusion proteins, and methods for using such fusion proteins to repair or regenerate damaged or diseased tissue.

### SUMMARY

The present invention is defined by the claims.

Compositions for use in methods of treating ischemic stroke are provided.

In some embodiments, the methods comprise administering by bolus injection to a subject in need thereof a pharmaceutical composition comprising a therapeutically effective amount of a chimeric protein and a pharmaceutically acceptable carrier, wherein the chimeric protein comprises, in order from N-terminus: (a) an activator domain comprising a variant of human insulin-like growth factor IGF-1, wherein the variant of IGF-1 comprises an amino acid sequence as set forth in SEQ ID NO: 2, (b) a half-life modulator comprising a variant of human serum albumin (HSA), wherein the variant of HSA comprises an amino acid sequence as set forth in SEQ ID NO: 15, and (c) a targeting domain comprising a variant of human Annexin 5 (AnxV), wherein the variant of AnxV comprises an amino acid sequence as set forth in SEQ ID NO: 10, optionally with a substitution to serine at position 315. Administration of the pharmaceutical composition results in at least one of the following: mitigation of oxidative damage to cells of the cerebral cortex, repair or acceleration of repair of blood brain barrier, reduction of oedema, reduction of infarct volume, reduction of blood brain barrier permeability, targeted stimulation of the phosphorylation of serine/threonine protein kinase B (AKT) pathway by selective activation of the IGF-1 receptor in cells of injured brain tissue, targeted delivery of pro-survival signals to injured brain tissue, increase in musculoskeletal coordination following stroke, improvement to consciousness following stroke, improvement to neurologic function following stroke, and improvement in motor function following stroke.

In some embodiments, the variant of IGF-1 decreases activation of the IGF-1 receptor relative to the wild-type IGF-1.

The variant of IGF-1 comprises E3R and Y31A substitutions relative to wild type human IGF-1.

The variant of human Annexin 5 comprises the amino acids 2-320 corresponding to wild type human Annexin 5 and comprises R63A, K70A, K101A, E138A, D139G, N160A, C316A substitutions relative to wild type human Annexin 5.

The variant of human serum albumin comprises the amino acids 26-609 corresponding to wild type human serum albumin and comprises C58S and N527Q substitutions relative to wild type human serum albumin.

In some embodiments, the chimeric protein is IGF1(E3R/Y31A)_lk7_HSA26-609(C58S/N527Q)_lk7_AnxV2-320(R63A/K70A/K101A/E138A/D139G/N160A/C316A).

In some embodiments, the linker lk7 comprises or consist of -Gly-Ser-Gly-Gly-Gly-Ser-Gly.

In some embodiments, the chimeric protein is selectively targeted to cells comprising a target molecule phosphatidylserine and exhibits activation of the IGF-1 receptor at least twice as strong on cells containing the target molecule compared to cells that do not contain the target molecule as measured by phosphorylation of serine/threonine protein kinase B (AKT).

In some embodiments, the method comprises administering the pharmaceutical composition within 72 hours of diagnosis of the ischemic stroke. In some embodiments, the method comprises administering the pharmaceutical composition within 48 hours of diagnosis of the ischemic stroke.

In some embodiments, the method comprises administering daily from about 0.01 mg/kg to about 20 mg/kg of the chimeric protein to the subject in need thereof.

In some embodiments, the method comprises administering a total dose of from about 5 mg/kg to about 20 mg/kg of the chimeric protein to the subject in need thereof over a period of 4 to 7 days. In some embodiments, the method comprises administering a total dose of from about 5 mg/kg to about 20 mg/kg of the chimeric protein to the subject in need thereof over a period of 7 days.

In some embodiments, the method comprises administering descending doses of the chimeric protein to the subject in need thereof. In some embodiments, the method comprises administering to the subject in need thereof a first dose comprising from about 2 mg/kg to about 6 mg/kg of the chimeric protein on day 1, and a dose comprising from about 1 mg/kg to about 2 mg/kg one each of the following days.

In some embodiments, the composition for use as described herein may be provided in a kit. The kit comprises a plurality of individual containers, each individual container comprising about 20 mg to about 1,000 mg of the chimeric protein.

### DESCRIPTION OF THE SEQUENCE LISTING

SEQ ID NO: 1 is the amino acid sequence of wild-type human IGF-1 (mature form).
SEQ ID NO: 2 is the amino acid sequence of a variant of wild-type human IGF-1 variant comprising E3R and Y31A substitutions.
SEQ ID NO: 3 is the amino acid sequence a variant of human IGF-1 (IGF-1 LONG).
SEQ ID NO: 4 is the amino acid sequence a variant of human IGF-1 (IGF1 E3R).
SEQ ID NO: 5 is the amino acid sequence of a variant of human IGF-1(IGF-1Des 1-3).
SEQ ID NO: 6 is the amino acid sequence of a variant of human IGF-1 (IGF-1 LR3).
SEQ ID NO: 7 is the amino acid sequence a variant of human IGF-1 (IGF1 R37X).
SEQ ID NO: 8 is the amino acid sequence of human a variant of human IGF-1 with deletion of residues 68-70 (IGF1 3X).
SEQ ID NO: 9 is the amino acid sequence of wild type human annexin A5 (AnxV).
SEQ ID NO: 10 is the amino acid sequence of a variant of wild-type human annexin 5 comprising the amino acids 2-320 of wild type annexin 5 and the R63A, K70A, K101A, E138A, D139G, N160A and C316A substitutions.
SEQ ID NO: 11 is the amino acid sequence of non-internalizing variant of human annexin A5 (ni-AnxV).
SEQ ID NO: 12 is the amino acid sequence of wild type Human Serum Albumin (HSA).
SEQ ID NO: 13 is the amino acid sequence of Human Serum Albumin variant mHSA (C34S, N503Q substitutions).
SEQ ID NO: 14 is the amino acid sequence of Human Serum Albumin variant mHSA7 (C34S, N503Q, E505G and V547A substitutions).
SEQ ID NO: 15 is the amino acid sequence of a variant human serum albumin comprising the amino acids 26-609 of wild type human serum albumin and the C58S and N527Q substitutions.
SEQ ID NO: 16 is the amino acid sequence of a peptide linker.
SEQ ID NO: 17 is the amino acid sequence of human transferrin (Tf).
SEQ ID NO: 18 is the amino acid sequence of Human Alpha Fetoprotein (AFP).
SEQ ID NO: 19 is the amino acid sequence of Human Vitamin D Binding Protein (VDBP).
SEQ ID NO: 20 is the amino acid sequence of Human Transthyretin (TTR).
SEQ ID NO: 21 is the amino acid sequence of a motif PASylation.
SEQ ID NO: 22 is the amino acid sequence of the albumin-binding domain human antibody (aldudAB).
SEQ ID NO: 23 is the amino acid sequence of a peptide linker lk7.
SEQ ID NO: 24 is the amino acid sequence of chimeric protein scp776.
SEQ ID NO: 25 is the amino acid sequence of chimeric protein IGF1(E3R/Y31A)_lk7_HSA26-609(C58S/N527Q)_lk7_AnxV2-320(R63A/K70A/K101A/E138A/D139G/N160A/C316S).

### BRIEF DESCRIPTION OF THE DRAWINGS

FIG. 1 is a graph showing that chimeric protein scp776 protects against oxidative neuronal cell death in vitro (percent released LDH readout).
FIG. 2 is a graph showing that chimeric protein scp776 protects against oxidative neuronal cell death in vitro (percent neuronal cell death readout).
FIG. 3 is a schematic representation of filament placement in the rat tMCAO model of acute ischemic stroke.
FIG. 4 is a graph showing that intravenous administration of chimeric protein scp776 protects blood brain barrier integrity after ischemic stroke in rats.
FIG. 5 is a graph showing that intravenous administration of chimeric protein scp776 reduces hemispheric swelling after ischemic stroke in rats.
FIG. 6 is a graph showing that intravenous administration of chimeric protein scp776 reduces infarct size after ischemic stroke in rats.
FIG. 7 is a graph showing that chimeric protein scp776 accumulates in rat brain tissue following ischemic stroke
FIG. 8 is a graph showing that chimeric protein scp776 treatment enhances recovery of function following ischemic stroke in rats.
FIG. 9 shows IGF-1 residues identified via application of an algorithm that considers solvent accessibility, hydrophobicity, and temperature factor. Represented by X are residue position that can be mutated.
FIG. 10 shows hydrophobic core positions of IGF-1.
FIG. 11A shows the neurological deficit score (NDS)- motor system score at 48 hours (day 2) for placebo treated and scp776 treated animals.
FIG. 11B shows the NDS- consciousness score at day 7 for placebo treated and scp776 treated animals.
FIG. 11C shows the NDS - musculoskeletal coordination score at day 1, 2, 3, 7, 10, and 14 for placebo treated (top curve) and scp776 treated (bottom curve) animals.
FIG. 11D shows the total NDS score at day 1, 2, 3, 7, 10, and 14 for placebo treated (top curve) and scp776 treated (bottom curve) animals.
FIG. 12 shows the results of the MRI analysis of the lesion size (mm²) at 8 hours, 72 hours and 14 days for placebo treated and scp776 treated animals.

### DETAILED DESCRIPTION

It is to be understood that the terminology used herein is for the purpose of describing particular embodiments of the disclosure only and is not intended to be limiting.

Unless defined otherwise, all technical and scientific terms used herein have the same meaning as commonly understood by one having ordinary skill in the art to which the disclosure pertains.

### Definitions

As used in this specification and the appended claims, the singular forms "a, "an" and "the" include plural referents unless the content clearly dictates otherwise.

The term "peptide," "polypeptide" and "protein" are used interchangeably to denote a sequence polymer of at least two amino acids covalently linked by an amide bond (also referred herein as peptide bond).

As used herein the term "target molecule" refers to any molecule that is associated with a tissue (e.g. "at risk", diseased or damaged tissue). A "target cell" is meant to be a cell to which a protein or targeting domain thereof can specifically bind.

"Binding" or "specific binding" are used interchangeably herein and indicates that a protein (or the targeting polypeptide domain thereof or the activator domain thereof) exhibits substantial affinity for a specific molecule (e.g., targeting domain exhibits substantial affinity for a target molecule, or an activator domain exhibits substantial affinity for a molecule associated with the surface of a cell such as a growth factor receptor) or a cell or tissue bearing the molecule and is said to occur when the protein (or the targeting polypeptide domain thereof or the activator domain thereof) has a substantial affinity for a specific molecule and is selective in that it does not exhibit significant cross-reactivity with other molecules.

"Identity," as known in the art, is a relationship between two or more polypeptide or protein sequences, as determined by comparing the sequences. In the art, "identity" also refers to the degree of sequence relatedness between polypeptides or proteins, as determined by the match between strings of such sequences. "Identity" can be readily calculated by any bioinformational methods known in the art.

The term "parent polypeptide" refers to a wild-type polypeptide and the amino acid sequence or nucleotide sequence of the wild-type polypeptide is part of a publicly accessible protein database (e.g., EMBL Nucleotide Sequence Database, NCBI Entrez, ExPasy, Protein Data Bank and the like).

The term "mutant polypeptide" or "polypeptide variant" refers to a form of a polypeptide, wherein its amino acid sequence differs from the amino acid sequence of its corresponding wild-type (parent) form, naturally existing form or any other parent form. A mutant polypeptide can contain one or more mutations, e.g., substitution, insertion, deletion, addition etc. ..which result in the mutant polypeptide. Generally, variants are overall closely similar, and, in many regions, identical to the reference polypeptide. As used herein, "variant" refers to a polypeptide, differing in sequence from a native protein but retaining at least one functional and/or therapeutic property thereof as described elsewhere herein or otherwise known in the art.

The term "corresponding to a parent polypeptide" is used to describe a polypeptide of the disclosure, wherein the amino acid sequence of the polypeptide differs from the amino acid sequence of the corresponding parent polypeptide only by the presence of at least one amino acid variation. Typically, the amino acid sequences of the variant polypeptide and the parent polypeptide exhibit a high percentage of identity. In one example, "corresponding to a parent polypeptide" means that the amino acid sequence of the variant polypeptide has at least about 50% identity, at least about 60%, at least about 70%, at least about 80%, at least about 85%, at least about 90%, at least about 91%, at least about 92%, at least about 93%, at least about 94%, at least about 95%, at least about 96%, at least about 97%, at least about 98% identity or at least about 99% identity to the amino acid sequence of the parent polypeptide. In another example, the nucleic acid sequence that encodes the variant polypeptide has at least about 50% identity, at least about 60%, at least about 70%, at least about 80%, at least about 85%, at least about 90%, at least about 91%, at least about 92%, at least about 93%, at least about 94%, at least about 95%, at least about 96%, at least about 97%, at least about 98% identity or at least about 99% identity to the nucleic acid sequence encoding the parent polypeptide.

The term "substantial identity" or "substantial similarity," as used herein, when referring to a nucleic acid or fragment thereof, indicates that when optimally aligned with appropriate nucleotide insertions or deletions with another nucleic acid (or its complementary strand), there is nucleotide sequence identity in at least about 95% to 99% of the sequence. The term "substantial identity" or "substantial similarity," as used herein, when referring to a protein or fragment thereof, indicates that when optimally aligned there is an amino acid sequence identity in at least about 95% to 99% of the sequence.

The term "damaged cell" or "damaged tissue," as used herein, means and includes biological cell or tissue; for example, but not limited to, neurons, glia or nervous tissue damaged or injured by, but not limited to, trauma or chemical insult, ischemic tissue, cell or tissue damaged by any means which results in interruption of normal blood flow to the tissue.

The term "therapeutically effective amount," as used herein, means the amount of the protein or agent that will elicit the biological or medical response of a tissue, system, animal or human that is being sought by the researcher, veterinarian, medical doctor or other clinician.

The term "pharmaceutically acceptable," or "physiologically acceptable" as used herein, means the carrier, diluent or excipient must be compatible with the other ingredients of the formulation and not deleterious to the recipient thereof.

The term "targeting moiety", "targeting domain," "targeting polypeptide" or "targeting module" are used herein interchangeably and refer to molecules that selectively localize the chimeric protein in a particular tissue or region of the body. The localization can be mediated by specific recognition of molecular determinants, molecular size of the targeting domain, ionic interactions, hydrophobic interactions and the like. As used herein, the terms "therapeutic moiety," "activator domain," "activator polypeptide", "signaling arm" and "effector module" are used herein interchangeably and refers to any agents useful for therapy and that are nontoxic, do not have a cytotoxic effect or are not detrimental to the cells. Such agents can include, but not limited to, growth factors.

### Chimeric Proteins

Chimeric proteins provided herein are capable of specific binding to two or more different specific molecules. In some embodiments, the chimeric protein comprises a targeting domain having a binding specificity to a first specific target molecule, an activator domain having a binding specificity to a second target molecule, and a half-life modulator.

In some aspects, the activator domain has a binding specificity to a tyrosine kinase receptor at the cell surface. In some embodiments, the binding of the activator to the tyrosine kinase receptor activates intracellular signaling pathways associated with cell survival. In some aspects, the activator domain has a binding specificity to a receptor that modulates/promotes tissue regeneration.

In some embodiments, the targeting domain serves to target the chimeric protein to a target cell or tissue while the activator domain serves to activate the intracellular signaling pathway associated with cell survival.

In some embodiments, the half-life modulator extends the half-life of the chimeric protein.

The chimeric proteins are fusion proteins having a targeting polypeptide connected or linked to a half-life modulator and to an activator polypeptide. The engineered proteins are chimeric proteins having a targeting polypeptide connected or linked to a half-life modulator and a growth factor or mutated growth factor.

In some embodiments, the mutated growth factor (IGF-1 variant) is engineered to reduce potency while retaining the ability to activate the cognate growth factor receptor.

The targeting domain is generally used to target the chimeric proteins to a target cell. In some embodiments the target cell is undergoing apoptosis. The binding of the targeting domain to its target molecule does not induce a significant biological effect in the target cell. The activator domain binds to a receptor on a cell surface. The binding of the activator domain to its receptor is intended to modulate a specific biological effect, such as, activate the intracellular signaling pathway associated with cell survival. In some embodiments, binding of the activator domain to its receptor is intended the positively regulate survival of the targeted cells or tissue. In particular, the activator domain of chimeric protein can promote survival signaling.

The *in vivo* activity of the chimeric protein can be assessed by detecting signaling changes in molecules that are regulated by the activator domain, including but not limited to cell surface receptor phosphorylation status or downstream mediators such as phospho-AKT or phospho-ERK (as detected by flow cytometry, immunofluorescence, ELISA, phospho-labeling, Western analysis of treated tissues, or any other methodology known in the art.) In some examples, a chimeric protein functions in vivo if it induces a significant (e.g., at least 10%, at least 20%, at least 30%, at least 40%, at least 50% or more) change in the level, functional activity, or phosphorylation of the regulated molecule detected by the assay.

### Activator Domain

For example only, the activator domain may be any polypeptide that detectably modulates the activity of a cellular network. The activator domain may be capable of activating signal transduction pathways by binding to a receptor at the surface a cell. Certain activator domains may be growth factor polypeptides, or any agonist of the receptor. It will be apparent that such modulation may be an increase in the activity of the cellular network such as induction of proliferation of cells, induction of cell growth, promotion of cell survival and/or inhibition of apoptosis.

An activator domain for a particular application may be selected based on the desired therapeutic outcome. For example only, to increase survival and neuroprotection, activator domains that comprise IGF-1 (or variant or fragment thereof), NRG1 (or variant or fragment thereof), FGF2 (or variant or fragment thereof), FGF9 (or variant or fragment thereof), CCL4 (or variant or fragment thereof), Hepatocyte Growth Factor (NK1 or fragment thereof), Vascular Endothelial Growth Factor A (or variant or fragment thereof), BMP2 (or variant or fragment thereof), may be used.

The activator domain comprises a change in the amino acid sequence, the three-dimensional structure of the protein, and/or the activity of the protein, relative to the wild-type form of the protein.

The activator domain comprises a growth factor having amino acid sequence modification relative to the wild-type growth factor (IGF-1) to decrease its binding to its natural receptor (IGF-1 receptor), to decrease its binding to binding proteins (IGF binding proteins) and/or decrease its activation of its natural receptor (IGF-1 receptor). In some embodiments, the activator domain is a growth factor having amino acid sequence modification that reduce (e.g., for about 1- 5%, 5-10%, 10%-20%, about 20%-40%, about 50%, about 40%-60%, about 60%-80%, about 80%-90%, 90-95%) the binding to its natural receptor (IGF-1 receptor).

A growth factor polypeptide detectably modulates activation of a growth factor receptor. In some embodiments, the activator domain of the bi-specific protein is a variant of IGF-1 that retains at least about 0.01 % of wild-type biological activity. In some embodiments, the activator domain of the bi-specific protein is a variant of IGF-1 that retain at least about 0.1 %, at least about 1%, at least about 10%, of wild-type biological activity. In some embodiments, the activator domain of the bi-specific protein is a variant of IGF-1 that retains between about 0.01% to about 0.1% of wild-type biological activity. In some embodiments, the activator domain of the bi-specific protein is a variant of IGF-1 that retains between about 0.01% to about 1% of wild-type biological activity. In some embodiments, the activator domain of the bi-specific protein is a variant of IGF-1 that retains between about 0.01% to about 10% of wild-type biological activity. In some embodiments, the activator domain of the bi-specific protein is a variant of IGF-1 that retains between about 0.1% to about 1% of wild-type biological activity. In some embodiments, the activator domain of the bi-specific protein is a variant of IGF-1 that retains between about 0.1% to about 10% of wild-type biological activity. In some embodiments, the activator domain of the bi-specific protein is a variant of IGF-1 that retains between about 01% to about 10% of wild-type biological activity. Biological activity as described herein can be determined by measuring activation of the corresponding growth factor receptor in appropriate cells. Activation may be assessed, for example, by measuring phosphorylation of receptor kinase or downstream effector proteins, such as, but not limited to, AKT, S6, ERK, JNK, mTOR, etc.

### Insulin-like growth factors (IGFs) and derivatives thereof

The insulin-like growth factors (IGFs) constitute a family of proteins having insulin-like and growth stimulating properties. The IGFs Human IGF-1 is a 70 amino acids basic peptide having the protein shown in SEQ ID NO: 1, respectively. IGF-1 and extracellular tyrosine kinase receptor (e.g. IGF-1 receptor) are important for cellular processes such as cell proliferation and survival. Binding of IGF-1 or variant thereof to the IGF-1 receptor stimulates kinase activity, leading to phosphorylation of multiple substrate, thereby initiating signaling cascades. The chimeric proteins disclosed herein can maintain the ability to signal through the extracellular IGF-1 receptor. The activator domain IGF-1 stimulates cell proliferation and survival through activation of the AKT pathway. Upon binding of IGF-I to the IGF-1 receptor, a tyrosine kinase, phosphorylates tyrosine residues on two major substrates, IRS-1 and Shc, which subsequently signal through the Ras/Raf and PI 3-kinase/AKT pathways.

The interaction of IGF-1 (and IGF-2) with the IGF-1 receptor is regulated by IGF binding Proteins (IGFBPs). All six IGFBPs (particularly IGFBP5) have been shown to inhibit IGF action, but in some instances a stimulatory effect has been observed. At least 99% of the IGF in the circulation is normally bound to IGFBPs.

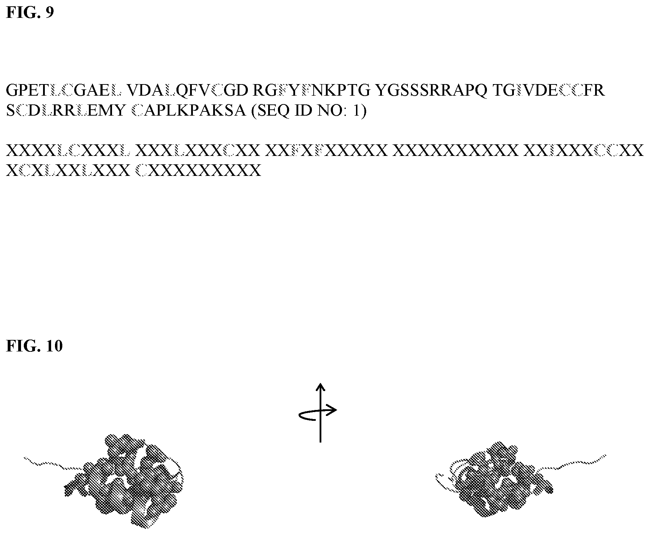

The activator domain is a variant of the human IGF-1. In some embodiments, the variant of IGF-1 or fragment thereof is capable of maintaining selectivity to the IGF-1 receptor. FIG. 9 shows residues identified via application of an algorithm that considers solvent accessibility, hydrophobicity, and temperature factor. Represented by X are residue position that can be mutated. Collectively, these residues represent the hydrophobic core of IGF-1. Hydrophobic core positions of IGF-1 are depicted as spheres (pdb model 1H02) at FIG. 10.

In some embodiments, the IGF-1 variant is modified to reduce binding to IGF-1 binding proteins (IGFBPs) relative to wild-type IGF-1 while maintaining its ability to activate the AKT pathway. In some embodiments, the IGF-1 variant can activate the IGF-1 receptor with a decreased potency for non-target cells, as assessed by pAKT EC50. EC50 is defined as the concentration needed to achieve the half maximal level of pAKT signaling.

The IGF-1 variant comprises the substitution Y31A. Also described herein are further exemplary IGF-1 variants. An exemplary IGF-1 variant may comprise one or more substitutions at position Y24, Y31 and Y60. An exemplary IGF-1 variant may comprise a single tyrosine substitution at position Y31, or Y24, or Y60. An exemplary IGF-1 variant may comprise a single tyrosine substitution at position Y24 and Y31, Y24 and Y60, Y31 and Y60, or Y24 and Y60. An exemplary IGF-1 variant may comprise one or more of the following substitutions, Y24L, Y31A, and Y60L relative to wild type IGF-1. For example, an IGF-1 variant may comprise the Y24L substitution and the Y31A substitution or the exemplary IGF-1 variant may comprise the Y24L substitution, the Y31A substitution and the Y60L. In some examples described herein, one or more tyrosine residues (Y24, Y31, Y60 or combinations thereof) may be substituted for a short aliphatic amino acid. In some examples, one or more tyrosine residues (Y24, Y31, Y60 or combinations thereof) can be substituted for a polar amino acid. In some examples, one or more tyrosine residues (Y24, Y31, Y60 or combinations thereof) can be substituted for leucine, alanine, isoleucine, serine, threonine or any other amino acid.

For example only, IGF-1 variants may comprise a substitution replacing Arg for a Glu, Lys, Met, Val, Ala, Leu, Ile, Gly, Ser, or Thr at the 3 position of the polypeptide. The present IGF-1 variant comprises a substitution replacing Arg for a Glu at the 3 position of the polypeptide (E3R).

the present IGF-1 variant comprises a substitution at the position 3 and 31. The present IGF-1 variant comprises E3R and Y31A substitutions. The activator domain has an amino acid sequence having SEQ ID NO: 2.

In some examples, the activator domain is a derivative of the human IGF-1 comprising one or more of the following modifications: a N-terminal 13-residue extension (IGF-1 LONG), a deletion of amino acids 1-3 (Des-1-3), a substitution replacing Arg for a Glu at the 3 position of the polypeptide (E3R), no Arginine at position 37 (R37X), a deletion of amino acids 68-70 (3X), an N-terminal 13-residue extension and a substitution replacing Arg for a Glu at the 3 position of the wild -type polypeptide (LR3), substitutions of one or more of tyrosine residues (Y24, Y31, Y60 or combinations thereof (e.g. Y24L, Y31A, Y60L substitutions or combinations thereof).

In some examples, the activator domain is variant of the human IGF-1 comprising a mutation (e.g. substitution, deletion) at one or more residues 24 to 37.

In some examples, the activator domain is a derivative of the human IGF-1 and comprises an N-terminal 13-residue extension (also referred as IGF-1 LONG, SEQ ID NO: 3), a mutation E3R (SEQ ID NO: 4) or a combination thereof (LONG E3R, also referred as LR3, SEQ ID NO: 6). In some embodiments, the IGF-1 variant comprises the E3R substitution, an N-terminal 13-residue extension, deletion of amino acids 1-3 ((Des1-3), SEQ ID NO: 5) or a combination thereof to decrease the binding of the activator domain to the IGF binding proteins which are present in the serum and other body fluid.

In some examples, the activator domain is a derivative of the human IGF-1 and comprises one or more of the following modifications: an N-terminal 13-residue extension (SEQ ID NO: 3), a deletion of amino acids 1-3 (SEQ ID NO: 5), a substitution replacing Arg for a Glu at the 3 position of the polypeptide (SEQ ID NO: 4), no Arginine at position 37 (R37X, SEQ ID NO: 7), a deletion of amino acids 68-70 (3X, SEQ ID NO: 8), or an N-terminal 13-residue extension and a substitution replacing Arg for a Glu at the 3 position of the wild - type polypeptide (SEQ ID NO: 6).

It is believed that the bi-specific proteins that contain the variant of IGF-1 described herein (e.g. E3R, IGF-1 LONG, IGF-1 LONG E3R (referred to as IGF-1(LR3)) or IGF1 Des1-3), have decreased affinity for IGF binding proteins relative to wild-type IGF-1. In some embodiments, the IGF-1 variants of the bi-specific proteins described herein can activate the signaling pathway while having a substantially decreased interaction with the IGF-1 binding proteins relative to wild-type IGF-1.

In some embodiments, the IGF-1 variant can be modified by glycosylation of one or more glycosylation site present in the IGF-1 variant.

In some embodiments, the chimeric proteins that contain the IGF-1 variants described herein have a potency for non-target cells that is less than wild-type IGF-1 for non-target cells.

Certain activator domains that bind to growth factor receptors are described herein in SEQ ID NOs: 1-8.

Additional peptide sequence modifications can be included, such as variations, deletions, substitutions or derivatizations of the amino acid sequence of the sequences disclosed herein, so long as the peptide has substantially the same activity or function as the unmodified peptides. Notably, a modified peptide will retain activity or function associated with the unmodified peptide, the modified peptide will generally have an amino acid sequence "substantially homologous" with the amino acid sequence of the unmodified sequence.

In some embodiments, the IGF-1 variant can have an amino acid sequence having at least about 85%, at least about 90%, at least about 91%, at least about 92%, at least about 93%, at least about 94%, at least about 95%, at least about 97%, at least about 98% identity or at least about 99% identity to the amino acid sequence provided in SEQ ID NO: 2. In some embodiments, the IGF-1 variant can have an amino acid sequence having from about 85% to about 90%, from about 90% to about 95%, from about 95% to about 98%, from about 98% identity to about 99% identity to the amino acid sequence provided in SEQ ID NO: 2. In some embodiments, the IGF-1 variant can have an amino acid sequence recited in SEQ ID NO: 2.

In some embodiments, the bi-specific protein comprises an activator domain having a growth factor variant that is selected to give the bi-specific protein at least an order of magnitude lower EC50 in damaged tissue than in healthy tissue. For example, the bi-specific protein domain comprises a growth factor variant and has an EC50 in damaged tissue that is at least 10 times lower, at least 15 times lower, at least 20 times lower, at least 25 times lower, at least 30 times lower, at least 35 times lower, at least 40 times lower, at least 45 times lower, at least 50 times lower, at least 55 times lower, at least 60 times lower, at least 65 times lower, at least 70 times lower, at least 75 times lower, at least 80 times lower, at least 85 times lower, at least 90 times lower, at least 95 times lower, at least 100 times lower, at least 110 times lower than the EC50 in healthy tissue.

In some embodiments, the bi-specific proteins that contain the IGF-1 variants have a half maximal effective concentration (EC50) that is lower in damaged tissue than in healthy tissue. In some embodiments, the bi-specific proteins that contain the IGF-1 variants have a half maximal effective concentration (EC50) that is at least 10 times lower, at least 15 times lower, at least 20 times lower, at least 25 times lower, at least 30 times lower, at least 35 times lower, at least 40 times lower, at least 45 times lower, at least 50 times lower, at least 55 times lower, at least 60 times lower, at least 65 times lower, at least 70 times lower, at least 75 times lower, at least 80 times lower, at least 85 times lower, at least 90 times lower, at least 95 times lower, at least 100 times lower, at least 110 times lower in damaged tissue than in healthy tissue.

In some embodiments, the chimeric proteins provided herein having such variant growth factors have a higher specificity to the damaged tissue targeted.

### Target molecules

In some aspects, target molecules are exposed or enriched on the exterior of a target cell. In some embodiments, the target molecule is associated with a damaged cell, early apoptotic or apoptotic cell, the target molecule being intracellular in a viable or undamaged cell and being exposed to the extracellular space in a damaged cell. Such molecules include, for example, molecules that are exposed in cells that undergo necrosis (such as DNA) or apoptosis (e.g., phosphatidylserine), myosin (including the tissue type-specific subtypes thereof), ICAM-1 or P-selectin. Yet in other embodiments, the target molecule is a molecule that is present or enriched at the surface of a diseased or dysfunctional cell or tissue as compared to the level detected in a healthy or functional cell or tissue. In some embodiments, the target cell is not a tumor or cancerous cell.

Cells are bounded by a plasma membrane (or cell membrane) comprising a lipid bilayer. The cell membrane may be considered to have a surface facing the cytosol (cytosolic side or interior of the cell) and a surface facing the exterior of the cell, or the extracellular space. Trans-bilayer movement of anionic phospholipids from the inner to the outer leaflet of the plasma membrane occurs during apoptosis. The anionic phospholipid-binding protein, such as Annexin A5, synaptotagmin I or lactadherin can be used to detect the presence of phosphatidylserine on the outer leaflet of the cell membrane. Phosphatidylserine is a phospholipid, that is usually restricted to the cytosolic side of the membrane in viable or undamaged cells, and that becomes exposed on the outer cell surface or to the extracellular space in damaged cells or apoptosis.

In some embodiments, the target molecule is an "ischemia-associated molecule". An "ischemia-associated molecule" is any molecule that is detected at a level that is significantly higher (e.g., at least 1.5 higher, at least 2-fold higher, at least 3-fold higher, at least 4-fold higher, at least 5-fold higher) following ischemia (which results in hypoxia) or hypoxia than in a cell of the same tissue that has not undergone an ischemic event (i.e., the molecule is specific to or enriched in the post-ischemic tissue). Ischemia occurs when there is insufficient blood flow to provide adequate oxygenation, which results in tissue hypoxia (reduced oxygen) or anoxia (absence of oxygen) as the most severe form of hypoxia, and ultimately tissue necrosis, and apoptosis.

### Targeting Domain

In some embodiments, the targeting domain has a specific binding affinity to a target molecule associated with a tissue (for example, an ischemia-associated molecule). In some embodiments, the targeting domain has a specific binding affinity for a target molecule presented on the surface of early apoptotic cells. In some embodiments, binding of the targeting domain to the target molecule does not have or does not modulate a biological activity. As used herein, "biological activity" refers to a defined, known activity performed by exposure of a molecule to a domain of the protein.

### Annexin A5 and variants thereof

The targeting domain comprises a variant of annexin. The annexin is Annexin A5 but, for example only, other annexins can equally be used. The targeting domain comprises a variant of human Annexin A5. A variant of Annexin A5 comprises at least one amino acid in at least one position in which this amino acid is not found in the parent wild type Annexin A5 polypeptide (SEQ ID NO: 9). In some examples, the Annexin A5 variant can have an amino acid sequence having at least about 85%, at least about 90%, at least about 95%, at least about 98% identity or at least about 99% identity to the amino acid sequence provided in SEQ ID NO: 9. In some examples, the Annexin A5 variant can comprise 50, 80, 100, 110, 200, 300, or more consecutive amino acid having at least about 85%, at least about 90%, at least about 95%, at least about 98% identity or at least about 99% identity to the amino acids in SEQ ID NO: 9.

In some embodiments, the variant of Annexin A5 is modified to substitute cysteine at position 315 (corresponding to C316) with serine to reduce dimer formation. As used herein, the term "corresponding to" is used to designate the position/identity of an amino acid residue in a polypeptide (e.g., Annexin A5). Those of ordinary skill will appreciate that, for purposes of simplicity, a canonical numbering system (based on wild-type Annexin A5) is utilized herein, so that an amino acid "corresponding to" a residue at position 316, for example, need not actually be the 316th amino acid in a particular amino acid chain but rather corresponds to the residue found at position 316 in a for example Annexin A5 before the post-translational removal of the N-terminal methionine; those of ordinary skill in the art readily appreciate how to identify corresponding amino acids. In particular, it is noted that the amino acid sequence of wild-type Annexin A5 (SEQ ID NO: 9) do not start with a Methionine as the Methionine residue is cleaved during processing.

In some embodiments, the variant of Annexin A5 has an amino acid sequences that has been mutated to reduce internalization of Annexin A5 or the chimeric protein comprising the variant of Annexin A5 into a cell while maintaining binding affinity to phosphatidylserine (PS). In some embodiments, the variant of Annexin A5 or the chimeric protein comprising the variant of Annexin A5 has a binding affinity to phosphatidylserine, and is not internalized into a cell or is internalized at a slower rate than wild-type annexin A5. In some examples, the targeting domain is a non-internalizing variant of Annexin A5, (also referred as ni-Annexin A5 or ni-AnxV, SEQ ID NO: 11). In some embodiments, the variant of Annexin A5 has an amino acid set forth in SEQ ID NO: 10. In some examples, the non-internalizing mutant of Annexin A5 has an amino acid sequence having at least about 85%, at least about 90%, at least about 95%, at least about 98% identity or at least about 99% identity to the amino acid sequence provided in SEQ ID NO: 11. In some examples, the non-internalizing mutant of Annexin A5 can have an amino acid sequence having from about 85% to about 90%, from about 90% to about 95%, from about 95% to about 98%, from about 98% to about 99% identity to the amino acid sequence provided in SEQ ID NO: 11. In some examples, the Annexin A5 variant can comprise 50, 80, 100, 110, 200, 300, or more consecutive amino acid of any one of amino acids in SEQ ID NO: 11. Any variation of Annexin A5 that results in substantially no internalization is envisioned.

It should be appreciated that the non-internalizing variant of annexin A5 can confer an extended half-life to the chimeric protein as compared to a chimeric protein that contains wild-type A5. In some embodiments, the variants of annexin A5 that results in substantially no internalization, or chimeric proteins containing variants of annexin A5 that results in substantially no internalization, can have an extended half-life of 1.1 to 1.2, 1.1 to 1.3, 1.1. to 1.4, 1.1 to 1.5, 1.1 to 1.6, 1.1 to 1.7, 1.1 to 1.8, 1.1 to 1.9, 1.1 to 2 or greater as compared to wild-type annexin A5, or chimeric proteins containing wild-type annexin A5.

The terms "non-internalizing" and "substantially no internalization," as used herein, refer to a lack of internalization of a substantial amount of the chimeric protein disclosed herein. For example, the phrase "substantially no internalization" will be understood as less than 50% of the chimeric protein being internalized by a cell to which the chimeric protein is bound, or less than 25% of the chimeric protein being internalized by a cell to which the chimeric protein is bound, or less than 10% of the chimeric protein being internalized by a cell to which the chimeric protein is bound, or less than 5% of the chimeric protein being internalized by a cell to which the chimeric protein is bound, or less than 3% of the chimeric protein being internalized by a cell to which the chimeric protein is bound, or less than 1 % of the chimeric protein being internalized by a cell to which the bi-specific protein is bound.

In some examples, the non-internalizing mutant of Annexin A5 can have an amino acid sequence having at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% identity or at least about 99% identity to human Annexin A5. In some examples, the non internalizing variant of Annexin A5 comprises a substitution at position 315 (corresponding to C316) wherein the cysteine residue is substituted with serine (Ser), alanine (Ala), leucine (Leu), phenylalanine (Phe), methionine (Met) or tryptophan (Trp). In some examples, the non-internalizing mutant of Annexin A5 can have an amino acid sequence having at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% identity or at least about 99% identity to human Annexin A5. In some examples, the non internalizing variant of Annexin A5 comprises a substitution at position 315 (corresponding to C316) wherein the cysteine residue is substituted with serine (Ser) or alanine (Ala). In some examples, the non-internalizing mutant of Annexin A5 can have an amino acid sequence having at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% identity or at least about 99% identity to human Annexin A5 modified to substitute cysteine at position 315 (corresponding to C316) with serine or alanine.

In some examples, Annexin A5 or Annexin A5 variants (for example variant having a substitution at position C316) are modified to comprise one or more substitutions at the following positions: R62, K69, K100, E137, D138, N159, L313 (corresponding to R63, K70, K101, E138, D139, N160, L314 relative to wild type human Annexin A5).

Annexin A5 variants described herein for example only may comprise:
R62A, R62E, R62D, R62M, R62L, R62I, R62Y (corresponding to R63A, R63E, R63D, R63M, R63L, R63I, R63Y relative to wild type human Annexin A5);
K69A, K69E, K69D, K69M, K69L, K69I, K69Y (corresponding to K70A, K70E, K70D, K70M, K70L, K70I, K70Y relative to wild type human Annexin A5);
E137A, E137K, E137R, E137M, E137L, E137I, E137Y (E138A, E138K, E138R, E138M, E138L, E138I, E138Y relative to wild type human Annexin A5);
D138G, D138K, D138R, D138M, D138L, D138I, D138Y (corresponding to D139G, D139K, D139R, D139M, D139L, D139I, D139Y relative to wild type human Annexin A5)
N159A, N160M, N160L, N160I, N160V, N160Y (corresponding to N160A, N160M, N160L, N160I, N160V, N160Y relative to wild type human Annexin A5)
L313E L313D, L313K, L313R, L313H, L313Q, L313N, L313Y (corresponding to L314E L314D, L314K, L314R, L314H, L314Q, L314N, L314Y relative to wild type human Annexin A5);
or any combinations of the foregoing.

In some examples, Annexin A5 or Annexin A5 variants comprise one or more substitutions at position D143 and/or E227. In some examples, Annexin A5 variant comprises:
D142G, D142A, D142K, or D142R (corresponding to D143G, D143A, D143K, or D143R) substitution, and/or
E226G, E226A, E226K, or E226R (corresponding to E227G, E227A, E227K, E227R) substitution

In some examples, Annexin A5 or Annexin A5 variants (for example having a substitution at C316, D143 and/or E227) are modified to comprise one or more of the following substitutions R62A, K69A, K100A, E137A, D138G, N159A, L313E (corresponding to R63A, K70A, K101A, E138A, D139G, N160A, L314E). For example, Annexin A5 having SEQ ID NO: 9 can be modified to have C315A or C315S substitution (corresponding to C316A or C316S relative to wild type Annexin A5) and one or more of the following substitutions R62A, K69A, K100A, E137A, D138G, N159A, L313E (corresponding to R63A, K70A, K101A, E138A, D139G, N160A, L314E relative to wild type Annexin A5).

In some examples, human Annexin A5 (SEQ ID NO: 9) are modified to comprise one or more of the following substitutions R62A, K69A, K100A, E137A, D138G, N159A, D143N, E227A, C315S or C315A (corresponding to R63A, K70A, K101A, E138A, D139G, D144N, N160A, E228A, C316S or C316A relative to wild type Annexin A5).

The present targeting domain comprises Annexin A5 which has been engineered to have R63A, K70A, K101A, E138A, D139G, N160A and C316A or C316S substitutions relative to wild type Annexin A5. For example, the targeting domain can have the amino acid sequence of SEQ ID NO: 10.

In some examples, the Annexin A5 variant comprises one, two, , three, four, five, six, seven, eight, nine, ten, eleven, twelve, thirteen, fourteen, fifteen, sixteen, seventeen, eighteen nineteen, twenty or more substitutions in different regions, in order to further decrease the internalization of the annexin in a cell. For example, the Annexin A5 variants may comprise R62A and K69A, R62A and K100A, R62A and E137A, R62A and D138G, R62A and N159A, R62A and K69A and K100A, R62A and K69A and E137A, R62A, K69A and K100A, R62A, K69A, K100A, and E137A etc...

The annexin variants described herein may further comprise one or more amino acid substitutions, deletions, or additions, wherein the amino acid substitutions, deletions, or additions do not substantially affect the ability of the Annexin A5 variant of the chimeric protein to bind to at least one phospholipid, such as PS.

For example only, native polypeptide can be used as targeting domains. It will be apparent, however, that portions of such native sequences and polypeptides having altered sequences may also be used, provided that such polypeptides retain the ability to bind the target molecule with an appropriate binding affinity (Kd) as described in more details below.

### Antibody targeting domain:

For example only, an anti-phosphatidylserine antibody may be used as a targeting domain. As used herein, term "antibody" includes but is not limited to: (i) a Fab fragment, a monovalent fragment consisting of the VL, VH, CL and CH1 domains; (ii) F(ab)2 and F(ab')2 fragments, a bivalent fragment comprising two Fab fragments linked by a disulfide bridge at the hinge region; (iii) a Fd fragment consisting of the VH and CH1 domains; (iv) a scFv fragment consisting of the VL and VH domains of a single arm of an antibody, (v) a dAb fragment which consists of a VH domain; and (vi) an isolated complementarity determining region (CDR). Such antibodies may be produced from intact antibodies using methods known in the art, or may be produced recombinantly, using standard recombinant DNA and protein expression technologies.

### Binding of Targeting domain

In some embodiments, the chimeric protein binds to the target molecule with a K_{d} of less than 10⁻⁶ M, preferably less than 10⁻⁷ M, 10⁻⁸ M, 10⁻⁹ M or 10⁻¹⁰ M.

### Half-Life Modulator

One skilled in the art would appreciate that proteins used in therapeutic applications may not exhibit optimal serum half-lives due to their relatively low molecular weight. In some therapeutic applications, it may therefore be desirable to extend the half-life of the proteins. To achieve accumulation of the chimeric protein to the diseased injured or damaged area of an organ, the chimeric protein is conjugated operatively associated or fused with a half-life modulator. Preferably, the half-life modulator is non-immunogenic polypeptide.

For example, short half-life is the most limiting attribute of wild-type growth factors as therapeutics. Intravenous administered IGF-1 has a serum half-life in humans of less than 1 hour. The extended half-life of chimeric proteins disclosed herein compared to IGF-1, for example, allows for 1) equivalent efficacy with less frequent dosing; 2) equivalent exposure at a lower dose; 3) lower Cmax at an equivalent exposure level, reducing the risk of Cmax-related toxicity.

In some embodiments, the half-life modulators can increase the *in vivo* half-life of the chimeric proteins. For example, the half-life of the chimeric proteins comprising the half-life modulator is about 1 hour, 2 hour, 3 hours, 4 hours, 5 hours, 6 hours or greater. For example, the half-life of the chimeric proteins can be about 8 hours or more when tested in cynomolgus monkey. In some embodiments, the half-life of the chimeric proteins comprising the half-life modulator is about 24 hours, or greater. In some embodiments, the half-life of the chimeric proteins comprising the half-life modulator is about a week or greater.

In some examples, the half-life modulator is non-immunogenic in humans.

In some examples, the half-life modulator is a polypeptide that interacts with cellular machinery that promote evasion of lysosomal degradation pathways (e.g. - FcRn receptor-mediated recycling).

The present half-life modulator is designed to extend the half-life of the chimeric protein through binding to serum components such as Human Serum Albumin (HSA). HSA is the most abundant protein in the blood and has a demonstrated safety in humans.

The half-life modulator comprises a HSA variant. In some examples, the half-life modulator comprises at least 100 consecutive amino acids that are at least 70%, 80%, 85%, 90%, 91%, 92%, 93%, 94%, 95%, 96%, 97%, 98%, or 99% identical to wild type human serum albumin amino acid sequence (wtHSA, SEQ ID NO: 12). In some examples, the half-life modulator comprises at least 200 consecutive amino acids that are at least 70%, 80%, 85%, 90%, 91%, 92%, 93%, 94%, 95%, 96%, 97%, 98%, or 99% identical to wild type human serum albumin amino acid sequence. In some examples, the half-life modulator comprises at least 300 consecutive amino acids that are at least 70%, 80%, 85%, 90%, 91%, 92%, 93%, 94%, 95%, 96%, 97%, 98%, or 99% identical to wild type human serum albumin amino acid sequence. In some examples, the half-life modulator comprises at least 400 consecutive amino acids that are at least 70%, 80%, 85%, 90%, 91%, 92%, 93%, 94%, 95%, 96%, 97%, 98%, or 99% identical to wild type human serum albumin amino acid sequence. In some examples, the half-life modulator comprises at least 500 consecutive amino acids that are at least 70%, 80%, 85%, 90%, 91%, 92%, 93%, 94%, 95%, 96%, 97%, 98%, or 99% identical to wild type human serum albumin amino acid sequence.

In some examples, the HSA variant can have one of more of the following substitutions:
cysteine C58 can be substituted, for example, with serine (C58S), alanine (C58A), asparagine (C58N), leucine (C58L), or glutamine (C58Q),
lysine K420 can be substituted for example, with glutamic acid (K420E), aspartic acid (K420D), a leucine (K420L) or methionine (K410M),
asparagine N527 can be substituted for example, with glutamine (N527Q), aspartic acid (N527D), histidine (N527H), or tyrosine (N527Y),
glutamic acid E505 can be substituted for example, with glycine (E505G), alanine (E505A), leucine (E505L), lysine (E505K), valine (E505V), isoleucine (E505I), methionine (E505M), or glutamine (E505Q),
valine V547 can be substituted for example, with alanine (V547A), glycine ( V547G), leucine (V547L), lysine (V547K), isoleucine (V547I), methionine (V547M),
asparagine N503 can be substituted for example, with a Glutamine (N527Q), aspartic acid (N503D), histidine (N503H), or tyrosine (N503Y), or glutamine (V547Q).

In some examples, the HSA variant can have amino acids 26-609 and have one of more of the following substitutions:
cysteine C58 can be substituted for example, with serine (C58S), alanine (C58A), asparagine (C58N), leucine (C58L), or glutamine (C58Q),
lysine K420 can be substituted for example, with glutamic acid (K420E), aspartic acid (K420D), a leucine (K420L) or methionine (K410M),
asparagine N527 can be substituted for example, with glutamine (N527Q), aspartic acid (N527D), histidine (N527H), or tyrosine (N527Y),
glutamic acid E505 can be substituted for example, with a glycine G (E505G), alanine (E505A), leucine (E505L), lysine (E505K), valine (E505V), isoleucine (E505I), methionine (E505M), or glutamine (E505Q),
valine V547 can be substituted for example, with an alanine (V547A), glycine ( V547G), leucine (V547L), lysine (V547K), isoleucine (V547I), methionine (V547M),
asparagine N503 and/or N527 can be substituted for example, with Glutamine (N503Q and/or N527Q), aspartic acid (N503D and/or N527D), histidine (N503H and/or N527H), or tyrosin (N503Y and/or N527Y).

In some examples, the HSA variant (referred herein as mHSA) may have the following substitutions: C34S, N503Q (SEQ ID NO: 13). In some examples, the HSA variant (referred herein as mHSA7) may have the following substitutions C34S, N503Q, E505G and V547A (SEQ ID NO: 14). The present HSA variant comprises amino acids 26-609 and the following substitutions C58S and N527Q (SEQ ID NO: 15).

In some examples, the asparagine at position 503 and/or 527 of HSA, which may be deamidated and decrease half-life, can be removed by the N503Q substitution and/or the N527Q. In some examples, the cysteine C34 of HSA may be substituted to serine or alanine (S or A) to remove the free cysteine and minimize alternate disulfide-bond formation. In some examples, the half-life modulator is a modified version of the domain III (mHSA_dIII) of a modified HSA with the N503Q substitution and an additional terminal glycine. Such a modified version retains the HSA property of binding to FcRn and increased serum half-life.

For example only, the half-life modulator may be a Fc domain of an antibody or a single chain constant fragment. In some examples, the half-life modulator comprises Fc regions of an immunoglobulin molecule (e.g. IgG). In some examples, the half-life modulator comprises at least 100 consecutive amino acids that are at least 70%, 80%, 85%, 90%, 91%, 92%, 93%, 94%, 95%, 96%, 97%, 98%, or 99% identical to a human Fc amino acid sequence. The Fc domain of an antibody has a natural capability to bind FcRn, resulting in an extended half-life. In some examples, the Fc domain of an antibody is engineered not to bind Fc(gamma)R In an example, the Fc domain is engineered to substitute N297 with Q (N297Q variant). In some examples, the half-life modulator is a monomeric variant form of Fc (scFc). For example, the subset of IgG heavy chain which naturally dimerizes to form Fc is hinge-CH2-CH3. In some examples, the Fc domain is engineered to form a single chain by linking the hinge-CH2-CH3 with a flexible linker such as GGGGSGGGGSGGGGSGGGGS (SEQ ID NO: 16) to create a hinge-CH2-CH3-linker-hinge-CH2-CH3 chain. In an example, the single chain Fc (scFc) is engineered to substitute N297 with Q and C220 with S (N297Q, C220S).

For example only, the half-life modulator may be a single chain variable fragment (scFv) of an antibody targeted to albumin or other circulating protein. In some examples, the half-life modulator comprises an amino acid sequence that is at least 70%, 80%, 85%, 90%, 91%, 92%, 93%, 94%, 95%, 96%, 97%, 98%, or 99% identical to scFv amino acid sequence directed to a specific antigen, such as, but not limited to, albumin. In some examples, the half-life modulator comprises at least 50, at least 100, at least 150, at least 200, at least 250 consecutive amino acids that are at least 70%, 80%, 85%, 90%, 91%, 92%, 93%, 94%, 95%, 96%, 97%, 98%, or 99% identical to a scFv amino acid sequence directed to a specific antigen, such as, but not limited to, albumin.

For example only, the half-life modulator may be transferrin such as human transferrin (Tf, SEQ ID NO: 17). In some examples, the half-life modulator comprises an amino acid sequence that is at least 70%, 80%, 85%, 90%, 91%, 92%, 93%, 94%, 95%, 96%, 97%, 98%, or 99% identical to human transferrin amino acid sequence. In some examples, the half-life modulator comprises at least 100, at least 200, at least 300, at least 400, at least 500, at least 600, at least 650 consecutive amino acids that are at least 70%, 80%, 85%, 90%, 91%, 92%, 93%, 94%, 95%, 96%, 97%, 98%, or 99% identical to a human transferrin amino acid sequence.

For example only, the half-life modulator may comprise at least 100 consecutive amino acids that are at least 70%, 80%, 85%, 90%, 91%, 92%, 93%, 94%, 95%, 96%, 97%, 98%, or 99% identical to wild type human alpha-fetoprotein amino acid sequence (AFP, SEQ ID NO: 18). In some examples, the half-life modulator comprises at least 100 consecutive amino acids that are about 70%, 80%, 85%, 90%, 91%, 92%, 93%, 94%, 95%, 96%, 97%, 98%, or 99% identical to wild type human alpha-fetoprotein (AFP) amino acid sequence. In some examples, the N-linked glycosylation site of the AFP is removed by the N251Q substitution.

For example only, the half-life modulator may comprise at least 100 consecutive amino acids that are at least 70%, 80%, 85%, 90%, 91%, 92%, 93%, 94%, 95%, 96%, 97%, 98%, or 99% identical wild-type vitamin D-binding protein amino acid sequence (VDBP, SEQ ID NO: 19). In some examples, the half-life modulator comprises at least 100 consecutive amino acids that are about 70%, 80%, 85%, 90%, 91%, 92%, 93%, 94%, 95%, 96%, 97%, 98%, or 99% identical wild-type vitamin D-binding protein (VDBP) amino acid sequence. In some examples, the N-linked glycosylation site of the VDBP can be removed by the N288Q or N288T substitution.

For example only, the half-life modulator may comprise at least 100 consecutive amino acids that are at least 70%, 80%, 85%, 90%, 91%, 92%, 93%, 94%, 95%, 96%, 97%, 98%, or 99% identical to wild-type human transthyretin amino acid sequence (TTR, SEQ ID NO: 20). In some examples, the half-life modulator comprises at least 100 consecutive amino acids that are about 70%, 80%, 85%, 90%, 91%, 92%, 93%, 94%, 95%, 96%, 97%, 98%, or 99% identical to wild type human transthyretin (TTR) amino acid sequence. In some examples, the transthyretin is modified to remove the N118 N-glycosylation site. In some examples, the half-life modulator is a monomeric form of TTR.

For example only, the half-life modulator may comprise at least 100 consecutive amino acids that are at least 70%, 80%, 85%, 90%, 91%, 92%, 93%, 94%, 95%, 96%, 97%, 98%, or 99% identical to a PASylation amino acid sequence. PASylation are proline-, alanine-, and/or serine-rich sequences that mimic PEGylation (see WO/2008/155134). In some examples, the half-life modulator comprises at least 100 consecutive amino acids that are about 70%, 80%, 85%, 90%, 91%, 92%, 93%, 94%, 95%, 96%, 97%, 98%, or 99% identical to a PASylation amino acid sequence. PASylation are proline-, alanine-, and/or serine-rich sequences that mimic PEGylation. Polypeptide stretches of proline, alanine, and/or serine form semi-structured three-dimensional domains with large hydrodynamic radius, thereby reducing clearance of fusion proteins. In some examples, the PASylation amino acid sequence is about 200, 300, 400, 500 or 600 amino acids long. For example, the PASylation is a 20 times repeat of the amino acid sequence ASPAAPAPASPAAPAPSAPA (SEQ ID NO: 21).

For example only, the half-life modulator may comprise the attachment of polyethylene glycol (PEG) chain or chains to the fusion proteins through chemical attachment either to the N- and/or C-terminus and/or to an amino acid side chain (e.g., PEG-maleimide attachment to cysteines). PEG chains form semi-structured three-dimensional domains with large hydrodynamic radius, thereby reducing clearance of fusion proteins.

For example only, the half-life modulator may comprise at least 100 consecutive amino acids that are at least 70%, 80%, 85%, 90%, 91%, 92%, 93%, 94%, 95%, 96%, 97%, 98%, or 99% identical to an albumin-binding domain human antibody (albudAb) amino acid sequence (SEQ ID NO: 22). In some examples, the half-life modulator comprises at least 100 consecutive amino acids that are about 70%, 80%, 85%, 90%, 91%, 92%, 93%, 94%, 95%, 96%, 97%, 98%, or 99% identical to an albumin-binding domain human antibody (albudAb) amino acid sequence. Albumin-binding domain antibodies can increase the fusion protein half-life by binding non-covalently to serum albumin (see WO2008/096158). In some examples, the albumin-binding domain human antibody is engineered to remove the C-terminal arginine to remove the Lys-Arg Kex2 protease site.

Such exemplary half-life modulators include those recited in any one of SEQ ID NOs: 12-15, 17-22.

In some examples, the half-life modulators can be modified to substitute the cysteine residues to serine or alanine residues to reduce the ability to form disulfide bonds.

In some embodiments, the targeting domain and activator domain can be joined via a half-life modulator. Accordingly, the half-life modulator can have two termini, an N-terminus and a C-terminus. In some embodiments, the half-life modulator is joined at one terminus via a peptide bond to the targeting polypeptide domain and is joined at the other terminus via a peptide bond to the activator domain. The half-life modulator is joined at the C-terminus to the targeting polypeptide domain and at the N-terminus to the activator domain. Yet, in other embodiments, the half-life modulator is joined at one of the termini of the bi-specific protein. For example, the half-life modulator can be joined at the N-terminus to the C-terminus of the activator domain.

### Peptide Linkers

In some embodiments, the activator domain, half-life modulator, and targeting domain are linked by peptide linker (e.g., from 2 to 40, 2-50, 2-100 amino acid residues) such that upon target recognition and engagement by the targeting domain, the presentation of the activator domain is optimized for binding to and activation of extracellular receptors on the surface of cells that present the target at a given surface density (e.g. - 5 x10² molecules / 1,000 Å2).

Targeted delivery of the activator domain for example IGF-1 for the activation of receptors on cells or tissues displaying a specific target requires appropriate presentation of both the activator domain and the targeting domain. In some embodiments, the flexibility of the linker is optimized for proper geometry of the engaged chimeric protein. Some of the principal determinants of the geometric constraints are the distances from the cell surface for the target and the receptor.

Additional optimization can be driven by the relative number of receptors and target molecules. At high ratios of Receptor : Target molecule, the engagement of both domains is reaction-limited. When the target molecule is more abundant than the receptor, the occupancy of both domains is diffusion-limited. Under the reaction-limit, optimal delivery of the activator domain is attained via short and rigid linkers. Under the diffusion limit, long and flexible linkers allow the activator domain to access a larger surface area. For cells with complex shapes (i.e. - bodies and neuronal processes) and receptor distributions, appropriate design of linker flexibility can enable precise targeting to sub-cellular regions.

In some embodiments, the peptide linker is present at the N-terminus, at the C-terminus or at both the N-terminus and the C-terminus of the half-life modulator at one or both ends. Suitable short connector polypeptides for use at the N-terminal end of the linker include, for example, dipeptides such as -Gly-Ser- (GS), -Gly-Ala- (GA) and -Ala-Ser- (AS). Suitable peptide linkers for use at the C-terminal end of the linker include, for example, dipeptides such as -Leu-Gln- (LQ) and -Thr-Gly- (TG). In some embodiments, the peptide linkers are longer than 2 amino acids. For example, the peptide linkers are 5, 6, 7, 8, 9, 10, 11, 12, 13, 14, 15, 16, 17, 18, 19 or 20 amino acids long or longer. In some embodiments, the peptide linkers are 20 or more 30 or more, 40 or more, 50 or more, 60 or more, 70 or more, 80 or more, 90 or more, 100 or more amino acids long. Preferably, such peptide linkers are flexible (for example glycine-rich) or structured (e.g., alpha-helix rich). In some embodiments, the linker comprises or consist of amino acids - Gly-Ser-Gly-Gly-Gly-Ser-Gly (SEQ ID NO: 23).

It will be apparent that elements in addition to those described above may optionally be included in the proteins provided herein. Such elements may be present for a variety of purposes, including to facilitate expression, preparation or purification of the bi-specific fusion protein, or to perform targeting functions.

### Representative chimeric protein

For example only, a representative bi-specific fusion protein may comprise (from N-terminal to C-terminal):
159... a targeting polypeptide domain comprising or consisting of a non-internalizing human annexin V variant (e.g., comprising or consisting of amino acids 2-320 of wt human Annexin 5 and a substitution at C316, R63, K40, K101, E138, D139, N160);
159... a linker peptide (e.g., - Gly-Ser-Gly-Gly-Gly-Ser-Gly);
159... a half-life modulator (e.g., HSA variant comprising or consisting of amino acids 26-609 of wt human HSA and comprising substitution at C58 and N527);
159... linker peptide (e.g., - Gly-Ser-Gly-Gly-Gly-Ser-Gly);
159... an activator domain comprising or consisting of an IGF-1 variant (e.g., comprising a substitution at E3 and Y31.

In some embodiments, the chimeric protein comprises or consists of IGF1(E3R/Y31A)_lk7_HSA26-609(C58S/N527Q)_lk7_AnxV2-320(R63A/K70A/K101A/E138A/D139G/N160A/C316A) (also referred herein as scp776). In some embodiments, the chimeric protein has an amino acid sequence as set forth in SEQ ID NO: 24.

In some embodiments, the chimeric protein comprises or consists of IGF1(E3R/Y31A)_lk7_HSA26-609(C58S/N527Q)_lk7_AnxV2-320(R63A/K70A/K101A/E138A/D139G/N160A/C316S). In some embodiments, the chimeric protein has an amino acid sequence as set forth in SEQ ID NO: 25.

### Nucleic acids

Described herein are polynucleotides encoding the chimeric proteins that may be in the form of RNA or in the form of DNA, which DNA includes cDNA and synthetic DNA. The DNA may be double-stranded or single-stranded. The coding sequences that encode the variants of the present disclosure may vary as a result of the redundancy or degeneracy of the genetic code.

### Pharmaceutical Compositions-Methods of Treatment

Pharmaceutical compositions for use in the treatment of ischemic stroke are provided. Injuries to brain due for example to reduced blood flow such as stroke, trauma, concussion, or neurodegenerative disorders can lead to neurologic deficit, loss of behavioral function or death. Stroke is one of the main causes of death in the Unites States. Stroke can be categorized into "ischemic stroke" (about 87%) and "hemorrhagic stroke" (about 10%). Ischemic stroke occurs when the blood supply to the brain is suddenly interrupted, generally caused by thrombus lodging or forming in one of the blood vessels supplying the brain. Hemorrhagic stroke happens when a blood vessel located the around the brain bursts leading to the leakage and accumulation of blood directly in the brain tissue. Interventional treatments must occur within hours of the onset of symptoms otherwise ischemia may lead to permanent neurologic deficit or death. Traumatic brain injury (TBI) leads mostly to a focal ischemia that occurs when an external force injures the brain. Mild and moderate TBIs lead mainly to brain contusions of different degrees causing edema associated brain ischemia. Moderate and severe TBIs lead to polytraumatic injuries such as vessel destruction, intracranial bleeding, brain tissue destruction, which can be associated with ischemic conditions in the affect brain regions.

Acute CNS injury in neurological and cognitive deficits. More than 65% of moderate to severe TBI patients report long-term problems with cognitive functioning. Such deficits can have a profound long-term effect on patients' outcomes.

Compositions for use in methods of treatment of acute CNS injury are described herein. The acute CNS injury is ischemic stroke. In some examples, the acute CNS injury is iatrogenic in nature. In some examples, methods for acute CNS injury neuroprotection are provided. In some examples, the method results in at least one or more of the following: improvement in neurological function, improvement in mental function, improvement in cognitive function, and increased survival rate. Measures of neurological function can be assessed with Rankin Score, modified Rankin Score, Barthel Index, modified Barthel Index, NIH stroke score, and clinical neurologic examinations.

Pharmaceutical compositions comprising a therapeutically effective amount of at least one chimeric protein as described herein, together with at least one physiologically acceptable carrier, are provided. Such compositions may be used for treating patients who are suffering from, or at risk for, tissue damage, in order to prevent tissue damage, or to repair or regenerate damaged tissue, for example nervous system tissue. The chimeric proteins and the pharmaceutical compositions described herein can have a neuroprotective action and prevent or halt neuronal death. Such patients include, for example, patients who have experienced an ischemic stroke. If desired, other active ingredients may also be included within the pharmaceutical composition, such as stem cells or other agents that facilitate repair of damaged tissue.

A "patient" or a "subject" is a mammal, preferably a human. The term "treating" (or "treat" or "treatment") means slowing, reducing, or reversing the progression or severity of a symptom, disorder, condition, or disease.

The term "therapeutically effective amount" refers to the amount or dose of chimeric proteins described herein which, upon single or multiple dose administration to a patient, provides the desired treatment.

In some embodiments, the compositions or chimeric proteins described herein localizes to the central nervous system (CNS) tissue(s) subjected to ischemic injury whether by traversing the blood brain barrier (BBB) or due to permeabilization of the blood brain barrier.

In some embodiments, the compositions or chimeric proteins described herein delivers pro-survival signals to neurons and glia of the subject.

In some embodiments, the pharmaceutical compositions or chimeric proteins described herein accelerates repair processes to the blood brain barrier of the subject in need thereof. In some embodiments, the pharmaceutical compositions or chimeric proteins described herein protects the blood brain barrier of the subject in need thereof.

In some embodiments, the pharmaceutical compositions or chimeric proteins described herein can be administered following the diagnosis of acute CNS injury. In some examples, acute CNS injury is acute spinal cord injury or acute brain injury. In some embodiments, the pharmaceutical compositions or chimeric proteins described herein is administered at the first presentation of neurovascular disease or acute CNS injury. In some embodiments, the pharmaceutical compositions or chimeric proteins described herein can be administered following the first imaging assessment that confirms occlusion of an arterial vessel that supplies blood to the principal organ of the CNS. In some embodiments, acute CNS injury can be diagnosed by clinical examination, neurological examination, imaging, biochemical markers indicative of acute CNS injury, or any diagnostic technology known in the art. In some embodiments, the acute CNS injury can be diagnosed using magnetic resonance imaging (MRI), computer tomography (CT) scan or a combination thereof. A MRI and CT imaging can show fractures, brain hemorrhage, hematomas, contusions or brain tissue swelling. Brain tissue swelling can also be assessed by using an intracranial pressure probe that is inserted through the skull.

In some embodiments, the compositions or chimeric proteins described herein can be administered to a subject in need thereof at the time of interventional reperfusion in the subject, wherein the subject suffers from thromboembolism.

In some embodiments, the pharmaceutical compositions or chimeric proteins described herein can be administered within 30 minutes of diagnosis, within 1 hour of diagnosis, within 2 hours of diagnosis, within 3 hours of diagnosis, within 4 hours of diagnosis, within 5 hours of diagnosis, within 6 hours of diagnosis, within 7 hours of diagnosis, within 8 hours of diagnosis, within 10 hours of diagnosis, within 12 hours of diagnosis, within 14 hours of diagnosis, within 16 hours of diagnosis, within 18 hours of diagnosis, within 20 hours of diagnosis, within 22 hours of diagnosis, within 24 hours of diagnosis, within 30 hours of diagnosis, within 36 hours of diagnosis, within 42 hours of diagnosis, within 48 hours of diagnosis, or within 72 hours of diagnosis.

In some embodiments, a first dose of the pharmaceutical composition comprising the chimeric proteins can be administered within 30 minutes of diagnosis, within 1 hour of diagnosis, within 2 hours of diagnosis, within 3 hours of diagnosis, within 4 hours of diagnosis, within 5 hours of diagnosis, within 6 hours of diagnosis, within 7 hours of diagnosis, within 8 hours of diagnosis, within 10 hours of diagnosis, within 12 hours of diagnosis, within 14 hours of diagnosis, within 16 hours of diagnosis, within 18 hours of diagnosis, within 20 hours of diagnosis, within 22 hours of diagnosis, within 24 hours of diagnosis, within 30 hours of diagnosis, within 36 hours of diagnosis, within 42 hours of diagnosis, within 48 hours of diagnosis, or within 72 hours of diagnosis.

In some embodiments, the pharmaceutical compositions or chimeric proteins described herein can be administered following therapies that increase oxygen delivery to the injured issued, and in combination with other therapeutics that have neuroprotection properties.

In some embodiments, the pharmaceutical compositions or the chimeric protein can be administered to a subject in need thereof to protect or treat tissue or organ degeneration. For example only, the pharmaceutical compositions may be used to treat central nervous system disorders such as, but not limited to, Acute Ischemic Stroke, Hemorrhagic Stroke, Transient Ischemic Attack, Iatrogenic Stroke, Traumatic Brain Injury (TBI), Concussion, acquired brain injury, spinal cord injury, subarachnoid hemorrhage, Amyotrophic Lateral Sclerosis (ALS), also known as Lou Gehrig's disease, Alzheimer's Disease (AD), Parkinson's Disease (PD), Huntington's Disease, Diabetic Neuropathy, and Spinal Muscular Atrophy. In some embodiments, the acute CNS injury is subarachnoid hemorrhage. In some embodiments, the acute CNS injury is iatrogenic in nature.

For example only, the pharmaceutical compositions may also be used to treat ocular disorders such as, but not limited to, Retinopathy, Macular Degeneration, Glaucoma, Cataract, and Acute Retinal Arterial Ischemia.

In some embodiments, the pharmaceutical compositions can be administered in combination with or as a co-medication to existing treatments known in the art (i.e., tissue plasminogen activators, urokinase plasminogen activators, etc.).

As used herein, the term "physiologically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the bi-specific fusion protein is administered. Physiologically acceptable carriers can be sterile liquids, such as water and oils, including those of petroleum, animal, vegetable or synthetic origin (e.g., peanut oil, soybean oil, mineral oil, or sesame oil). Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water and ethanol. The pharmaceutical composition, if desired, can also contain minor amounts of wetting or emulsifying agents, or pH buffering agents.

In some embodiments, the pharmaceutical composition is a liquid formulation formulated for intravenous (IV) injection. In some embodiments, the composition is formulated for IV bolus injection.

In some embodiments, the pH of the pharmaceutical composition is from 7 to 8, for example about 7.5. In some embodiments, the pharmaceutical composition comprises in an alkalinizing agent, such as tromethamine or dibasic sodium phosphate. In some embodiments, the alkalinizing can be at a concentration of 10 to 50 mM, for example 20 mM. In some embodiments, the pharmaceutical composition comprises a surfactant. In some embodiments, the surfactant is a non-ionic surfactant such as polysorbate 80 or polysorbate 20 may be present in a concentration of 0.01%, 0.02%. 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, or 1% (w/v). In some embodiments, the pharmaceutical composition comprises sucrose. In some embodiments, the sucrose may be present in a concentration of 1%, 2%. 3%, 4%, 5%, 6%, 7%, 8%, 9%, 10% (w/v).

In some embodiments, the pharmaceutical composition comprising the chimeric protein comprises alkalinizing agent, a surfactant, sucrose or a combination thereof.

The pharmaceutical compositions of the disclosure may contain pharmaceutically acceptable auxiliary substances as required to approximate physiological conditions, such as pH adjusting and buffering agents, tonicity adjusting agents, wetting agents, detergents, preservatives and the like.

In some embodiments, the pharmaceutical composition comprises from about 1 to about 100 g/l chimeric protein, from about 10 mM to about 50 mM tromethamine, from about 2 to about 15 % (w/v) sucrose, from about 0.001% to about 0.04% (w/v) Polysorbate 80, at pH 7.5

In some embodiments, the chimeric protein (from about 1 to about 100 g/l) is formulated in 20 mM tromethamine, 7.5% sucrose, and 0.02% polysorbate 80 at pH 7.5 (via addition of HCl).

In some embodiments, the liquid composition is sterilized by conventional sterilization techniques, or sterile filtered. In some embodiments, the liquid composition is in a vial.

In some embodiments, the pharmaceutical composition is administered intravenously or intraarterially to a subject in need thereof via bolus injection. A bolus injection comprises, e.g., fast intravenous injection, for example less than 10 seconds (or less than 20, 30, 40, 50, 60 second), or intravenous infusion over less than approximately 3 minutes, 4 minutes, 5 minutes, 6 minutes, 7 minutes, 8 minutes, 9 minutes or 10 minutes. In some embodiments, the pharmaceutical composition is administered intravenously to a subject in need thereof. In some embodiments, the composition is administered as an I.V. push. In some embodiments, the administration is over 5 min. or less, 4 min. or less, 3 min. of less, 2 min. or less, 1 min or less (for example 50 s, 40 s, 30 s, 20 s or any administration time therebetween). In other embodiments, the composition is administered as a slow I.V. injection.

In some embodiments, the pharmaceutical composition is administered intravenously to a subject using a syringe injection pump at a rate of 0.5 - 25 mL/min.

In some embodiments, the pharmaceutical composition is administered intraarterially to a subject using a syringe injection pump at a rate of 0.05 - 10 mL/min.

In some embodiments, the pharmaceutical composition is administered intrathecally to a subject using techniques known in the art.

In some embodiments, a therapeutically effective amount generally is in the range of about 0.01 mg/kg to about 100.0 mg/kg per dose. In some embodiments, a therapeutically effective amount of a protein disclosed herein can be, e.g., about 0.01 mg/kg to about 0.1 mg/kg per dose, 0.01 mg/kg to about 1 mg/kg per dose, 0.01 mg/kg to about 10 mg/kg per dose, 0.01 mg/kg to about 100.0 mg/kg per dose, 0.1 mg/kg to about 1 mg/kg per dose, 0.1 mg/kg to about 10 mg/kg per dose, 0.1 mg/kg to about 100 mg/kg per dose, 1 mg/kg to about 100 mg/kg per dose or 10 mg/kg to about 100 mg/kg per dose.

In some embodiments, a therapeutically effective amount generally is in the range of about 0.01 mg/kg to about 20.0 mg/kg per dose. In some embodiments, a therapeutically effective amount cam be range from about 0.01 mg/kg to about 10.0 mg/kg per dose.

In some embodiments, a therapeutically effective amount of a protein disclosed herein can be from about 0.01 mg/kg to about 0.02 mg/kg per dose, about 0.01 mg/kg to about 0.03 mg/kg per dose, about 0.01 mg/kg to about 0.04 mg/kg per dose, about 0.01 mg/kg to about 0.05 mg/kg per dose, about 0.01 mg/kg to about 0.06 mg/kg per dose, about 0.01 mg/kg to about 0.07 mg/kg per dose, about 0.01 mg/kg to about 0.08 mg/kg per dose, about 0.01 mg/kg to about 0.09 mg/kg per dose, about 0.01 mg/kg to about 0.1 mg/kg per dose, about 0.01 mg/kg to about 0.2 mg/kg per dose, about 0.01 mg/kg to about 0.3 mg/kg per dose, about 0.01 mg/kg to about 0.4 mg/kg per dose, about 0.01 mg/kg to about 0.5 mg/kg per dose, about 0.01 mg/kg to about 0.6 mg/kg per dose, about 0.01 mg/kg to about 0.7 mg/kg per dose, about 0.01 mg/kg to about 0.8 mg/kg per dose, about 0.01 mg/kg to about 0.9 mg/kg per dose, about 0.01 mg/kg to about 1 mg/kg per dose, about 0.01 mg/kg to about 2 mg/kg per dose, about 0.01 mg/kg to about 3 mg/kg per dose, about 0.01 mg/kg to about 4 mg/kg per dose, about 0.01 mg/kg to about 5 mg/kg per dose, about 0.01 mg/kg to about 6 mg/kg per dose, about 0.01 mg/kg to about 7 mg/kg per dose, about 0.01 mg/kg to about 8 mg/kg per dose, about 0.01 mg/kg to about 9 mg/kg per dose, about 0.01 mg/kg to about 10 mg/kg per dose, about 0.01 mg/kg to about 11 mg/kg per dose, about 0.01 mg/kg to about 12 mg/kg per dose, about 0.01 mg/kg to about 13 mg/kg per dose, about 0.01 mg/kg to about 14 mg/kg per dose, about 0.01 mg/kg to about 15 mg/kg per dose, about 0.01 mg/kg to about 16 mg/kg per dose, about 0.01 mg/kg to about 17 mg/kg per dose, about 0.01 mg/kg to about 18 mg/kg per dose, about 0.01 mg/kg to about 19 mg/kg per dose, about 0.01 mg/kg to about 20 mg/kg per dose.

In some embodiments, a therapeutically effective amount of a protein disclosed herein can be from about 0.01 mg/kg to about 0.05 mg/kg per dose, about 0.05 mg/kg to about 0.1 mg/kg per dose, about 0.1 mg/kg to about 0.5 mg/kg per dose, about 0.05 mg/kg to about 1 mg/kg per dose, about 1 mg/kg to about 2 mg/kg per dose, about 2 mg/kg to about 3 mg/kg per dose, about 3 mg/kg to about 4 mg/kg per dose, about 4 mg/kg to about 5 mg/kg per dose, about 5 mg/kg to about 6 mg/kg per dose, about 6 mg/kg to about 7 mg/kg per dose, about 7 mg/kg to about 8 mg/kg per dose, about 8 mg/kg to about 9 mg/kg per dose, about 9 mg/kg to about 10 mg/kg per dose, about 10 mg/kg to about 11 mg/kg per dose, about 11 mg/kg to about 12 mg/kg per dose, about 12 mg/kg to about 13 mg/kg per dose, about 13 mg/kg to about 14 mg/kg per dose, about 14 mg/kg to about 15 mg/kg per dose, about 15 mg/kg to about 16 mg/kg per dose, about 16 mg/kg to about 17 mg/kg per dose, 17 mg/kg to about 18 mg/kg per dose, about 18 mg/kg to about 19 mg/kg per dose, about 19 mg/kg to about 20 mg/kg per dose.

In some embodiments, a therapeutically effective amount of a protein disclosed herein can be about 0.01 mg/kg per dose, about 0.02 mg/kg per dose, about 0.03 mg/kg per dose, about 0.04 mg/kg per dose, about 0.05 mg/kg per dose, about 0.06 mg/kg per dose, about 0.07 mg/kg per dose, about 0.08 mg/kg per dose, about 0.09 mg/kg per dose, about 0.1 mg/kg per dose, about 0.2 mg/kg per dose, about 0.3 mg/kg per dose, about 0.4 mg/kg per dose, about 0.5 mg/kg per dose, about 0.6 mg/kg per dose, about 0.7 mg/kg per dose, about 0.8 mg/kg per dose, about 0.9 mg/kg per dose, about 1 mg/kg per dose, about 1.1 mg/kg per dose, about 1.2 mg/kg per dose, about 1.3 mg/kg per dose, about 1.4 mg/kg per dose, about 1.5 mg/kg per dose, about 1.6 mg/kg per dose, about 1.7 mg/kg per dose, about 1.8 mg/kg per dose, about 1.9 mg/kg per dose, about 2 mg/kg per dose, about 2.1 mg/kg per dose, about 2.2 mg/kg per dose, about 2.3 mg/kg per dose, about 2.4 mg/kg per dose, about 2.5 mg/kg per dose, about 2.6 mg/kg per dose, about 2.7 mg/kg per dose, about 2.8 mg/kg per dose, about 2.9 mg/kg per dose, about 3 mg/kg per dose, about 3.1 mg/kg per dose, about 3.2 mg/kg per dose, about 3.3 mg/kg per dose, about 3.4 mg/kg per dose, about 3.5 mg/kg per dose, about 3.6 mg/kg per dose, about 3.7 mg/kg per dose, about 3.8 mg/kg per dose, about 3.9 mg/kg per dose, about 4 mg/kg per dose, about 4.1 mg/kg per dose, about 4.2 mg/kg per dose, about 4.3 mg/kg per dose, about 4.4 mg/kg per dose, about 4.5 mg/kg per dose, about 4.6 mg/kg per dose, about 4.7 mg/kg per dose, about 4.8 mg/kg per dose, about 4.9 mg/kg per dose, about 5 mg/kg per dose, about 5.1 mg/kg per dose, about 5.2 mg/kg per dose, about 5.3 mg/kg per dose, about 5.4 mg/kg per dose, about 5.5 mg/kg per dose, about 5.6 mg/kg per dose, about 5.7 mg/kg per dose, about 5.8 mg/kg per dose, about 5.9 mg/kg per dose, about 6 mg/kg per dose, about 6.1 mg/kg per dose, about 6.2 mg/kg per dose, about 6.3 mg/kg per dose, about 6.4 mg/kg per dose, about 6.5 mg/kg per dose, about 6.6 mg/kg per dose, about 6.7 mg/kg per dose, about 6.8 mg/kg per dose, about 6.9 mg/kg per dose, about 7 mg/kg per dose, about 7.1 mg/kg per dose, about 7.2 mg/kg per dose, about 7.3 mg/kg per dose, about 7.4 mg/kg per dose, about 7.5 mg/kg per dose, about 7.6 mg/kg per dose, about 7.7 mg/kg per dose, about 7.8 mg/kg per dose, about 7.9 mg/kg per dose, about 8 mg/kg per dose, about 8.1 mg/kg per dose, about 8.2 mg/kg per dose, about 8.3 mg/kg per dose, about 8.4 mg/kg per dose, about 8.5 mg/kg per dose, about 8.6 mg/kg per dose, about 8.7 mg/kg per dose, about 8.8 mg/kg per dose, about 8.9 mg/kg per dose, about 9 mg/kg per dose, about 9.1 mg/kg per dose, about 9.2 mg/kg per dose, about 9.3 mg/kg per dose, about 9.4 mg/kg per dose, about 9.5 mg/kg per dose, about 9.6 mg/kg per dose, about 9.7 mg/kg per dose, about 9.8 mg/kg per dose, about 9.9 mg/kg per dose, about 10 mg/kg per dose, about 11 mg/kg per dose, about 12 mg/kg per dose, about 13 mg/kg per dose, about 14 mg/kg per dose, about 15 mg/kg per dose, about 16 mg/kg per dose, about 17 mg/kg per dose, about 18 mg/kg per dose, about 19 mg/kg per dose, about 20 mg/kg per dose.

In some embodiments, a therapeutically effective amount generally is in the range of about 1 mg/kg to about 10.0 mg/kg per dose. In some embodiments, an effective amount of a protein disclosed herein can be, e.g., about 1 mg/kg to about 10 mg/kg per dose, 1 mg/kg to about 9 mg/kg per dose, 1 mg/kg to about 8 mg/kg per dose, 1 mg/kg to about 7 mg/kg per dose, 1 mg/kg to about 6 mg/kg per dose, 1 mg/kg to about 5 mg/kg per dose, 1 mg/kg to about 4 mg/kg per dose, 1 mg/kg to about 3 mg/kg per dose or 1 mg/kg to about 2 mg/kg per dose.

Dosing can be single dosage or cumulative (serial dosing), and can be readily determined by one skilled in the art. For instance, treatment of a nervous system disorder may comprise a one-time administration of an effective dose of the pharmaceutical composition disclosed herein. As a non-limiting example, an effective dose of the composition disclosed herein can be administered once to a patient, e.g., as a single injection or bolus. Alternatively, treatment of a nervous system disorder may comprise multiple administrations of an effective dose of the pharmaceutical composition disclosed herein carried out over a range of time periods, such as, e.g., four times daily, three times daily, twice daily, daily, once every few days, weekly, monthly or yearly. As a non-limiting example, a combination disclosed herein can be administered once or twice weekly to a patient. The timing of administration can upon such factors as the severity of the patient's symptoms. For example, an effective dose of the composition disclosed herein can be administered to a patient once a month for an indefinite period of time, or until the mammal no longer requires therapy.

In some embodiments, a therapeutically effective amount generally is in the range of about 0.01 mg/kg to about 200.0 mg/kg per day. In some embodiments, an effective amount of a protein disclosed herein can be, e.g., .g., about 0.01 mg/kg to about 0.1 mg/kg per day, 0.01 mg/kg to about 1 mg/kg per day, 0.01 mg/kg to about 10 mg/kg per day, 0.01 mg/kg to about 100.0 mg/kg per day, 0.01 mg/kg to about 200.0 mg/kg per day, 0.1 mg/kg to about 1 mg/kg per day, 0.1 mg/kg to about 10 mg/kg per day, 0.1 mg/kg to about 100 mg/kg per day, 0.1 mg/kg to about 200 mg/kg per day, 1 mg/kg to about 100 mg/kg per day, 1 mg/kg to about 200 mg/kg per day, 10 mg/kg to about 100 mg/kg per day or 10 mg/kg to about 100 mg/kg per day.

In some embodiments, a therapeutically effective amount generally is in the range of about 0.01 mg/kg to about 20.0 mg/kg per day. In some embodiments, a therapeutically effective amount generally is in the range of about 0.01 mg/kg to about 10.0 mg/kg per day.. In some embodiments, a therapeutically effective amount generally is in the range of about 1 mg/kg to about 20.0 mg/kg per day. In some embodiments, a therapeutically effective amount generally is in the range of about 1 mg/kg to about 10.0 mg/kg per day. In some embodiments, an effective amount of a protein disclosed herein can be, e.g., about 1 mg/kg to about 10 mg/kg per day, 1 mg/kg to about 9 mg/kg per day, 1 mg/kg to about 8 mg/kg per day, 1 mg/kg to about 7 mg/kg per day, 1 mg/kg to about 6 mg/kg per day, 1 mg/kg to about 5 mg/kg per day, 1 mg/kg to about 4 mg/kg per day, 1 mg/kg to about 3 mg/kg per day or 1 mg/kg to about 2 mg/kg per day.

In some embodiments, a therapeutically effective amount of a chimeric protein disclosed herein can be from about 0.01 mg/kg to about 0.02 mg/kg per day, about 0.01 mg/kg to about 0.03 mg/kg per day, about 0.01 mg/kg to about 0.04 mg/kg per day, about 0.01 mg/kg to about 0.05 mg/kg per day, about 0.01 mg/kg to about 0.06 mg/kg per day, about 0.01 mg/kg to about 0.07 mg/kg per day, about 0.01 mg/kg to about 0.08 mg/kg per day, about 0.01 mg/kg to about 0.09 mg/kg per day, about 0.01 mg/kg to about 0.1 mg/kg per day, about 0.01 mg/kg to about 0.2 mg/kg per day, about 0.01 mg/kg to about 0.3 mg/kg per day, about 0.01 mg/kg to about 0.4 mg/kg per day, about 0.01 mg/kg to about 0.5 mg/kg per day, about 0.01 mg/kg to about 0.6 mg/kg per day, about 0.01 mg/kg to about 0.7 mg/kg per day, about 0.01 mg/kg to about 0.8 mg/kg per day, about 0.01 mg/kg to about 0.9 mg/kg per day, about 0.01 mg/kg to about 1 mg/kg per day, about 0.01 mg/kg to about 2 mg/kg per day, about 0.01 mg/kg to about 3 mg/kg per day, about 0.01 mg/kg to about 4 mg/kg per day, about 0.01 mg/kg to about 5 mg/kg per day, about 0.01 mg/kg to about 6 mg/kg per day, about 0.01 mg/kg to about 7 mg/kg per day, about 0.01 mg/kg to about 8 mg/kg per day, about 0.01 mg/kg to about 9 mg/kg per day, about 0.01 mg/kg to about 10 mg/kg per day, about 0.01 mg/kg to about 11 mg/kg per day, about 0.01 mg/kg to about 12 mg/kg per day, about 0.01 mg/kg to about 13 mg/kg per day, about 0.01 mg/kg to about 14 mg/kg per day, about 0.01 mg/kg to about 15 mg/kg per day, about 0.01 mg/kg to about 16 mg/kg per day, about 0.01 mg/kg to about 17 mg/kg per day, about 0.01 mg/kg to about 18 mg/kg per day, about 0.01 mg/kg to about 19 mg/kg per day, or about 0.01 mg/kg to about 20 mg/kg per day.

In some embodiments, a therapeutically effective amount of a chimeric protein disclosed herein can be from about 0.01 mg/kg to about 0.05 mg/kg per day, about 0.05 mg/kg to about 0.1 mg/kg per day, about 0.1 mg/kg to about 0.5 mg/kg per day, about 0.05 mg/kg to about 1 mg/kg per day, about 1 mg/kg to about 2 mg/kg per day, about 2 mg/kg to about 3 mg/kg per day, about 3 mg/kg to about 4 mg/kg per day, about 4 mg/kg to about 5 mg/kg per day, about 5 mg/kg to about 6 mg/kg per day, about 6 mg/kg to about 7 mg/kg per day, about 7 mg/kg to about 8 mg/kg per day, about 8 mg/kg to about 9 mg/kg per day, about 9 mg/kg to about 10 mg/kg per day, about 10 mg/kg to about 11 mg/kg per day, about 11 mg/kg to about 12 mg/kg per day, about 12 mg/kg to about 13 mg/kg per day, about 13 mg/kg to about 14 mg/kg per day, about 14 mg/kg to about 15 mg/kg per day, about 15 mg/kg to about 16 mg/kg per day, about 16 mg/kg to about 17 mg/kg per day, 17 mg/kg to about 18 mg/kg per day, about 18 mg/kg to about 19 mg/kg per day, or about 19 mg/kg to about 20 mg/kg per day.

In some embodiments, a therapeutically effective amount of a chimeric protein disclosed herein can be about 0.01 mg/kg per day, about 0.02 mg/kg per day, about 0.03 mg/kg per day, about 0.04 mg/kg per day, about 0.05 mg/kg per day, about 0.06 mg/kg per day, about 0.07 mg/kg per day, about 0.08 mg/kg per day, about 0.09 mg/kg per day, about 0.1 mg/kg per day, about 0.2 mg/kg per day, about 0.3 mg/kg per day, about 0.4 mg/kg per day, about 0.5 mg/kg per day, about 0.6 mg/kg per day, about 0.7 mg/kg per day, about 0.8 mg/kg per day, about 0.9 mg/kg per day, about 1 mg/kg per day, about 1.1 mg/kg per day, about 1.2 mg/kg per day, about 1.3 mg/kg per day, about 1.4 mg/kg per day, about 1.5 mg/kg per day, about 1.6 mg/kg per day, about 1.7 mg/kg per day, about 1.8 mg/kg per day, about 1.9 mg/kg per day, about 2 mg/kg per day, about 2.1 mg/kg per day, about 2.2 mg/kg per day, about 2.3 mg/kg per day, about 2.4 mg/kg per day, about 2.5 mg/kg per day, about 2.6 mg/kg per day, about 2.7 mg/kg per day, about 2.8 mg/kg per day, about 2.9 mg/kg per day, about 3 mg/kg per day, about 3.1 mg/kg per day, about 3.2 mg/kg per day, about 3.3 mg/kg per day, about 3.4 mg/kg per day, about 3.5 mg/kg per day, about 3.6 mg/kg per day, about 3.7 mg/kg per day, about 3.8 mg/kg per day, about 3.9 mg/kg per day, about 4 mg/kg per day, about 4.1 mg/kg per day, about 4.2 mg/kg per day, about 4.3 mg/kg per day, about 4.4 mg/kg per day, about 4.5 mg/kg per day, about 4.6 mg/kg per day, about 4.7 mg/kg per day, about 4.8 mg/kg per day, about 4.9 mg/kg per day, about 5 mg/kg per day, about 5.1 mg/kg per day, about 5.2 mg/kg per day, about 5.3 mg/kg per day, about 5.4 mg/kg per day, about 5.5 mg/kg per day, about 5.6 mg/kg per day, about 5.7 mg/kg per day, about 5.8 mg/kg per day, about 5.9 mg/kg per day, about 6 mg/kg per day, about 6.1 mg/kg per day, about 6.2 mg/kg per day, about 6.3 mg/kg per day, about 6.4 mg/kg per day, about 6.5 mg/kg per day, about 6.6 mg/kg per day, about 6.7 mg/kg per day, about 6.8 mg/kg per day, about 6.9 mg/kg per day, about 7 mg/kg per day, about 7.1 mg/kg per day, about 7.2 mg/kg per day, about 7.3 mg/kg per day, about 7.4 mg/kg per day, about 7.5 mg/kg per day, about 7.6 mg/kg per day, about 7.7 mg/kg per day, about 7.8 mg/kg per day, about 7.9 mg/kg per day, about 8 mg/kg per day, about 8.1 mg/kg per day, about 8.2 mg/kg per day, about 8.3 mg/kg per day, about 8.4 mg/kg per day, about 8.5 mg/kg per day, about 8.6 mg/kg per day, about 8.7 mg/kg per day, about 8.8 mg/kg per day, about 8.9 mg/kg per day, about 9 mg/kg per day, about 9.1 mg/kg per day, about 9.2 mg/kg per day, about 9.3 mg/kg per day, about 9.4 mg/kg per day, about 9.5 mg/kg per day, about 9.6 mg/kg per day, about 9.7 mg/kg per day, about 9.8 mg/kg per day, about 9.9 mg/kg per day, about 10 mg/kg per day, about 11 mg/kg per day, about 12 mg/kg per day, about 13 mg/kg per day, about 14 mg/kg per day, about 15 mg/kg per day, about 16 mg/kg per day, about 17 mg/kg per day, about 18 mg/kg per day, about 19 mg/kg per day, or about 20 mg/kg per day.

In some embodiments, the effective dose is administered day to the subject in need thereof over a period of 2 days, 3 days, 4 days, 5 days, 6 days, 7 days, 8 days, 9 days, 10 days, 11 days, 12 days, 13 days, 14 days or more. In some embodiments, the effective dose is administered day to the subject having an acute CNS injury over a period of a minimum 2 days to a period of 14 days, for example 4, 5, 6, 7 days.

In some embodiments, the effective dose is administered day to the subject having neurovegetative disease over a period of a minimum 2 days to at least 1 month, 2 months, 3 months, 4 months, 5 months, 6 months, 7 months, 8 months, 9 months, 10 months, 11 months, 1 year, 2 years, 3 years, 4 years, 5 years, 6 years, 7 years, 8 years, 9 years, 10 years, 11 years, 12, years, 13 years, 14 years, 15 years, 16, years, 17 years, 18 years, 19 years or 20 years.

In some embodiments, the effective amount is administered once a day (or every 24 hours) to the subject in need thereof. In some embodiments, the effective amount is administered once a day to the subject in need thereof over a period of 2 days, 3 days, 4 days, 5 days, 6 days, 7 days, 8 days, 9 days, 10 days, 11 days, 12 days, 13 days, 14 days or more. In some embodiments, the effective amount is administered once a day to the subject in need thereof over a period of up to 2 days, up to 3 days, up to 4 days, up to 5 days, up to 6 days, up to 7 days, up to 8 days, up to 9 days, up to 10 days, up to 11 days, up to 12 days, up to 13 days, up to 14 days. In some embodiments, the effective amount is administered once a day to the subject in need thereof over a period of 7 days. In some embodiments, the effective amount is administered two, three or more times a day to the subject in need thereof. In some embodiments, the effective amount is administered two, three or more times a day to the subject in need thereof over a period of 2 days, 3 days, 4 days, 5 days, 6 days, 7 days, 8 days, 9 days, 10 days, 11 days, 12 days, 13 days, 14 days or more. In some embodiments, the effective amount is administered two, three or more times a day to the subject in need thereof over a period of up to 2 days, up to 3 days, up to 4 days, up to 5 days, up to 6 days, up to 7 days, up to 8 days, up to 9 days, up to 10 days, up to 11 days, up to 12 days, up to 13 days, up to 14 days. In some embodiments, the effective amount is administered two, three or more times a day to the subject in need thereof over a period of 7 days.

In some embodiments, a total dose of about 5 to 100 mg/kg of the chimeric proteins described herein is administered over a period of 4 days, 5 days, 6 days, or 7 days. In some embodiments, a total dose of about 5 to 100 mg/kg of the chimeric proteins described herein is administered over a period of 7 days. In some embodiments, a total dose of about 5 to 90 mg/kg of the chimeric proteins described herein is administered over a period of 4 days, 5 days, 6 days, or 7 days. In some embodiments, a total dose of about 5 to 90 mg/kg of the chimeric proteins described herein is administered over a period of 7 days. In some embodiments, a total dose of about 5 to 80 mg/kg of the chimeric proteins described herein is administered over a period of 4 days, 5 days, 6 days, or 7 days. In some embodiments, a total dose of about 5 to 80 mg/kg of the chimeric proteins described herein is administered over a period of 7 days. In some embodiments ,a total dose of about 5 to 70 mg/kg of the chimeric proteins described herein is administered over a period of 4 days, 5 days, 6 days, or 7 days. In some embodiments, a total dose of about 5 to 70 mg/kg of the chimeric proteins described herein is administered over a period of 7 days. In some embodiments, a total dose of about 5 to 60 mg/kg of the chimeric proteins described herein is administered over a period of 4 days, 5 days, 6 days, or 7 days. In some embodiments, a total dose of about 5 to 60 mg/kg of the chimeric proteins described herein is administered over a period of 7 days. In some embodiments, a total dose of about 5 to 50 mg/kg of the chimeric proteins described herein is administered over a period of 4 days, 5 days, 6 days, or 7 days. In some embodiments a total dose of about 5 to 50 mg/kg of the chimeric proteins described herein is administered over a period of 7 days. In some embodiments, a total dose of about 5 to 40 mg/kg of the chimeric proteins described herein is administered over a period of 4 days, 5 days, 6 days, or 7 days. In some embodiments, a total dose of about 5 to 40 mg/kg of the chimeric proteins described herein is administered over a period of 7 days. In some embodiments, a total dose of about 5 to 30 mg/kg of the chimeric proteins described herein is administered over a period of 4 days, 5 days, 6 days, or 7 days. In some embodiments, a total dose of about 5 to 30 mg/kg of the chimeric proteins described herein is administered over a period of 7 days. In some embodiments, a total dose of about 5 to 20 mg/kg of the chimeric proteins described herein is administered over a period of 4 days, 5 days, 6 days, or 7 days. In some embodiments, a total dose of about 5 to 20 mg/kg of the chimeric proteins described herein is administered over a period of 7 days. In some embodiments, a total dose of about 5 to 20 mg/kg of the chimeric proteins described herein is administered over a period of 4 days.

In some embodiments, a total dose of about 100 to 500 mg/kg of the chimeric proteins described herein is administered over a period of 4 days, 5 days, 6 days, or 7 days. In some embodiments, a total dose of about 100 to 500 mg/kg of the chimeric proteins described herein is administered over a period of 7 days. In some embodiments, a total dose of about 100 to 400 mg/kg of the chimeric proteins described herein is administered over a period of 4 days, 5 days, 6 days, or 7 days. In some embodiments a total dose of about 100 to 400 mg/kg of the chimeric proteins described herein is administered over a period of 7 days. In some embodiments, a total dose of about 100 to 300 mg/kg of the chimeric proteins described herein is administered over a period of 4 days, 5 days, 6 days, or 7 days. In some embodiments, a total dose of about 100 to 300 mg/kg of the chimeric proteins described herein is administered over a period of 7 days. In some embodiments, a total dose of about 100 to 200 mg/kg of the chimeric proteins described herein is administered over a period of 4 days, 5 days, 6 days, or 7 days. In some embodiments, a total dose of about 100 to 200 mg/kg of the chimeric proteins described herein is administered over a period of 7 days. In some embodiments, a total dose of about 100 to 150 mg/kg of the chimeric proteins described herein is administered over a period of 4 days, 5 days, 6 days, or 7 days. In some embodiments, a total dose of about 100 to 150 mg/kg of the chimeric proteins described herein is administered over a period of 7 days. In some embodiments, a total dose of about 140 mg/kg of the chimeric proteins described herein is administered over a period of 4 days, 5 days, 6 days, or 7 days. In some embodiments, a total dose of about 140 mg/kg of the chimeric proteins described herein is administered over a period of 7 days.

In some embodiments, the treatment regimen comprises a descending dosage regimen. In descending dosage regimen, the amount of the dose of the chimeric protein of the disclosure administered in the subject is reduced over the course of the duration of the treatment. In some embodiments, the dose administered on the second day is lower than the dose administered the second day. In other embodiments, the dose administered on the second day is the same than the dose administered the second day, and the dose administered the third day is lower than the dose administered the second day. In some embodiments, the treatment regimen provides for a total dose of about 2 mg/kg to about 200 mg/kg, of about 2 mg/kg to about 20 mg/kg, of about 2 mg/kg to about 10 mg/kg, of about 100 mg/kg to about 200 mg/kg, of about 100 mg/kg to about 150 mg/kg over a predetermined period of time (e.g. 4, 5, 6, or 7 days). In some embodiments, the treatment regimen provides for a total dose of about 5 mg/kg, of about 10 mg/kg, or of about 20 mg/kg over a predetermined period of time (e.g. 4, 5, 6, or 7 days).

In some embodiments, a first daily dose is administered the first day, a second daily dose that comprises from about 85% to about 95% of the amount of the chimeric protein present in the first dose is administered on day 2, a third daily dose that comprises from about 65% to about 85% of the amount of the chimeric protein present in the second dose and an amount that is lower than the second dose is administered on day 3, a fourth dose that comprises from about 45% to about 65% of the amount of the chimeric protein present in the first dose and an amount that lower than the third dose is administered on day 4, a fifth dose that comprises from about 35% to about 45% of the amount of the chimeric protein present in the first dose and an amount that lower than the fourth dose is administered on day 5, a sixth dose that comprises from about 25% to about 35% of the amount of the chimeric protein present in the first dose and an amount that lower than the fifth dose is administered on day 6, and a seventh dose that comprises from about 15% to about 25% of the amount of the chimeric protein present in the first dose and an amount that lower than the sixth dose is administered on day 7. In some embodiments, the descending treatment regimen comprises administering a first daily dose on day 1, a second daily dose corresponding to about 90% of the first daily dose on day 2, a third daily dose corresponding to about 70% of the first daily dose on day 3, a fourth daily dose corresponding to about 50% of the first daily dose on day 4, a fifth daily dose corresponding to about 40% of the first daily dose on day 5, a sixth daily dose corresponding to about 30% of the first daily dose on day 6 and a seventh daily dose corresponding to about 20% of the first daily dose on day 7.

In some embodiments, a first daily dose is administered the first day, a second daily dose that comprises from about 85% to about 95% (e.g. about 90%) of the amount of the chimeric protein present in the first dose is administered on day 2, a third daily dose that comprises from about 65% to about 85% (e.g. about 70%) of the amount of the chimeric protein present in the second dose and an amount that is lower than the second dose is administered on day 3, a fourth dose that comprises from about 45% to about 65% (e.g. about 50%) of the amount of the chimeric protein present in the first dose and an amount that lower than the third dose is administered on day 4.

In some embodiments, a first daily dose administered the first day and the second daily dose administered on the second day are the same, a third daily dose comprising from about 65% to about 90% (e.g. about 75%) of the amount of the chimeric protein present in the first dose is administered on day 3, a fourth daily dose that comprises from about 45% to about 65% (e.g. about 50%) of the amount of the chimeric protein present in the second dose and an amount that is lower than the second dose is administered on day 4.

In some embodiments, the treatment regimen or descending treatment regimen provides a total dose of from about 2 mg/kg to about 20 mg/kg over a period of 7 days. In some embodiments, the treatment regimen provides a total dose of from about 2 mg/kg to about 20 mg/kg over a period of 4 days. In some embodiments, the treatment regimen or descending treatment regimen provides a total dose of from about 2 mg/kg to about 10 mg/kg over a period of 7 days. In some embodiments, the treatment regimen or descending treatment regimen provides a total dose of from about 2 mg/kg to about 10 mg/kg over a period of 4 days. In some embodiments, the treatment regimen or descending treatment regimen provides a total dose of about 5 mg/kg, about 10 mg/kg, or about 20 mg/kg over a period of 7 days. In some embodiments, the treatment regimen or descending treatment regimen provides a total dose of about 5 mg/kg, about 10 mg/kg, or about 20 mg/kg over a period of 4 days.

In some embodiments, the treatment regimen comprises a 5 days course of intravenous administration of the chimeric protein, such as scp776. In some embodiments, the treatment regimen comprises a 7 days course of intravenous administration of the chimeric protein, such as scp776. In some embodiments, the treatment regimen comprises a 4 days course of intravenous administration of the chimeric protein, such as scp776. In some embodiments, the treatment regimen comprises a 3 days course of intravenous administration of the chimeric protein, such as scp776. In some embodiments, the treatment regimen comprises a 2 days course of intravenous administration of the chimeric protein, such as scp776. In some embodiments, the treatment regimen comprises administering intravenously a first dose at from about 2 mg/kg to about 6 mg/kg on day 1, and one dose of about 1 mg/kg to about 2 mg/kg one each of the following days. In some embodiments, the first and second dose are the same (e.g. 2 mg/kg), and the third dose is lower than the first dose. For example, the first dose can be 2 mg/kg, the second dose can be 2 mg/kg, the third dose can be 1.5 mg/kg and the fourth dose can be 1 mg/kg, in a 4 days treatment course. In some embodiments, the second dose is lower than the first dose and the third dose is lower than the second dose etc... For example, the first dose can be 2 mg/kg, the second dose can be 1.8 mg/kg, the third dose can be 1.4 mg/kg and the fourth dose can be 1 mg/kg, in a 4 days treatment course. In some embodiments, the second dose is lower than the first dose and the third dose is the same as the second dose, etc... For example, the course can comprise a first dose of about 5.2 mg/kg on day 1, and one dose of about 1.3 mg/kg on day 2, days 3, day 4 and day 5, if the course is a 5 days course.

In some embodiments, the course of treatment comprises administration of the effective amount over 2 days, 3 days, 4 days, 5 days, 6 days, 7 days, 8 days, 9 days, 10 days, 11 days, 12 days, 13 days, 14 days or more. In some embodiments, the course of treatment comprises administration of the effective amount over 7 days. In some embodiments, the course of treatment comprises administration of the effective amount over 4 days. In some embodiments, the course of treatment comprises administration of the effective amount over consecutive days. In some embodiments, the course of treatment comprises administration of the effective amount every day, every 2nd day, every 3rd day or every 4th day.

In some embodiments, the subject in need thereof treated with the chimeric protein or pharmaceutical compositions described herein is continuously infused with dextrose solution to sustain euglycemia. In some embodiments, the dextrose solution comprises from about 5% (w/v) dextrose to about 50% (w/v) dextrose. In some embodiments, the dextrose solution is in sterile water or normal saline solution. For example, the subject in need thereof can be administered i.e., 5% Dextrose in normal saline or 10% Dextrose in sterile water to sustain euglycemia. In some embodiments, the subject in need thereof is administered the chimeric protein or pharmaceutical compositions described herein via bolus injection or intrathecally and is administered a dextrose solution by continuous infusion. In some embodiments, the subject is infused with the dextrose solution for 48 hours. In some embodiments, the subject is infused with the dextrose solution up to the end of the treatment with the chimeric protein. In some embodiments, the subject is infused with the dextrose solution up to 24 hours after the end of the treatment with the chimeric protein. For example, the subject can be treated for 4 days with the pharmaceutical composition described herein and the subject can be infused for up to 4 or 5 days with the dextrose solution.

In some embodiments, the human dose regimen can be calculated based on allometric scaling of the Non-Human Primate to Human dose regimen.

An estimate of the human equivalent dose (HED) to the efficacious dosing regimen can be obtained via allometric scaling (USDHHS, FDA, CDER, Guidance for Industry, 2005). Allometric scaling treats the problem of differential metabolic rates between species by applying a correction factor based on body surface area to the dose of interest in a given species. Allometric scaling is most frequently used in estimating safe starting doses for first-in-human studies, but it is also commonly applied in translating effective doses from animals to man. The FDA-recommended correction factor for converting dosages in 3 kg rhesus monkeys to humans is 3.1 (USDHHS, FDA, CDER, Guidance for Industry, 2005). In some embodiments, the estimated HED of the efficacious dose regimen comprises an initial dose of 16 mg/kg ÷ 3.1 = 5.2 mg/kg, followed by additional doses at 24 hour intervals of 4 mg/kg ÷ 3.1 = 1.3 mg/kg.

In some embodiments, the pharmaceutical compositions described herein can further include one or more additional bioactive or therapeutic agents or components to aid in the treatment of damaged tissue or cells and/or facilitate the tissue regenerative process. In some embodiments, the additional therapeutic agent is a thrombolytic agent. In some embodiments, the thrombolytic agent is recombinant tissue plasminogen activator (rt-PA). In some embodiments, the additional therapeutics agent comprise a neuroprotective agent. In some embodiments, the additional therapeutic agent comprises or consists of Glucocorticoids, FK506, Cyclosporin, Sirolimus, nerinetide, oxygen carrying therapeutics (perfluorocarbons) or the like.

In some embodiments, the method comprises administering two or more (e.g. two, there or more) pharmaceutical compositions. The different pharmaceutical compositions may be administered to the subject in any order and in any suitable interval. For example, in some embodiments, the one or more some embodiments compositions are administered simultaneously or near simultaneously. In some embodiments, the method comprises a staggered administration of the two or more some embodiments compositions, where a first some embodiments composition is administered and a second some embodiments composition administered at some later time point. Any suitable interval of administration which produces the desired therapeutic effect may be used.

In certain embodiments, the method has an additive effect, wherein the overall effect of the administering a combination of therapeutic agents or procedures is approximately equal to the sum of the effects of administering each therapeutic agent or procedure alone. In other embodiments, the method has a synergistic effect, wherein the overall effect of administering a combination of therapeutic agents or procedures is greater than the sum of the effects of administering each therapeutic agent or procedure alone.

In some embodiments, the pharmaceutical compositions and chimeric proteins described herein, when administered to a subject having an acute CNS injury, result in at least one of more of the following: mitigation of oxidative damage to cells of the cerebral cortex, repair or acceleration of repair of blood brain barrier, reduction of oedema, reduction of infarct volume, reduction in blood brain barrier permeability, targeted delivery of pro-survival signals to injured brain tissue, increase in musculoskeletal coordination following stroke, improvements to consciousness following stroke, improvements to neurologic function following stroke, and increase in motor function following stroke.

In some embodiments, the pharmaceutical compositions and chimeric proteins described herein, when administered to a subject having a neurodegenerative disease, result in improvement in motor functions and decrease in disease symptoms, such as decrease in tremors in subjects having PD, decreases in autonomic dysfunction in subjects having ALS, or decrease in AD symptoms such as memory loss and confusion in subjects having AD.

In some embodiments, a therapeutically effective amount of the pharmaceutical composition comprising the chimeric protein reduces at least one symptom associated with a nervous system disorder by, e.g., at least 10%, at least 20%, at least 30%, at least 40%, at least 50%, at least 60%, at least 70%, at least 80%, at least 90% or at least 100%. In some embodiments, a therapeutically effective amount of the chimeric protein reduces at least one symptom associated with a nervous system disorder by, e.g., at least 10%, at least 20%, at least 30%, at least 40%, at least 50%, at least 60%, at least 70%, at least 80%, at least 90% or at least 100%. In some embodiments, a therapeutically effective amount of the chimeric protein disclosed herein reduces at least one symptom associated with a nervous system disorder by, e.g., about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%. In some embodiments, a therapeutically effective amount of the chimeric protein reduces at least one symptom associated with a nervous system disorder for, e.g., at least one week, at least one month, at least two months, at least three months, at least four months, at least five months, at least six months, at least seven months, at least eight months, at least nine months, at least ten months, at least eleven months, or at least twelve months.

In some embodiments, a therapeutically effective amount of the pharmaceutical composition comprising the chimeric protein results in an improvement of at least one neurological or cognitive function by, e.g., at least 10%, at least 20%, at least 30%, at least 40%, at least 50%, at least 60%, at least 70%, at least 80%, at least 90% or at least 99% or about 100%. In some embodiments, a therapeutically effective amount of the chimeric protein results in an improvement of at least one neurological or cognitive function by, e.g., at least 10%, at least 20%, at least 30%, at least 40%, at least 50%, at least 60%, at least 70%, at least 80%, at least 90% or at least 100%. In some embodiments, a therapeutically effective amount of the chimeric protein disclosed herein results in an improvement of at least one neurological or cognitive function by, e.g., about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%. In some embodiments, a therapeutically effective amount of the chimeric protein results in an improvement of at least one neurological or cognitive function for, e.g., at least one week, at least one month, at least two months, at least three months, at least four months, at least five months, at least six months, at least seven months, at least eight months, at least nine months, at least ten months, at least eleven months, or at least twelve months.

In some embodiments, administration of the effective amount to a subject having ischemic stroke results in a significant improvement in neurologic function as assessed by a decrease in the Neurologic Deficit Scoring (NDS) such as musculoskeletal coordination, consciousness, motor system; or improvement as assessed by any other neurologic function scale known in the art (e.g. NIH stroke scale, modified Rankin Scale, modified Barthel Index, or Glasgow coma scale, MDS-Unified Parkinson's Disease Rating Scale, or Hunt-Hess Classification).

In some embodiments, administration of the effective amount to a subject having ischemic stroke results in a significant reduction in lesion volume, perfusion deficit, blood brain barrier permeability, or any other imaging assessment of injury severity known in the art (e.g., Fisher scale). In some embodiments, the reduction is by at least 10%, at least 20%, at least 30%, at least 40%, at least 50%, at least 60%, at least 70%, at least 80%, at least 90% or at least 99% or about 100%.

In some embodiments, administration of the effective amount to a subject having ischemic stroke results in a significant improvement in cognitive function as assessed by for example, but not limited to, NIH stroke score, modified Rankin Score, and/or modified Barthel Index.

In some examples, administration of the effective amount to a subject having a neurodegenerative disease results in a significant improvement in cognitive function as assessed by, for example, but not limited to, USPDRS, SCAG, and ALS FRS.

Compositions for use in methods of treatment of ischemic stroke are provided. The methods comprise administering a therapeutically effective amount of the composition or chimeric proteins described herein by bolus injection to a subject in need thereof, wherein administration results in at least one of more of the following: mitigation of oxidative damage to cells of the cerebral cortex, repair or acceleration of repair of blood brain barrier, reduction of oedema, reduction of infarct volume, reduction of blood brain barrier permeability, targeted delivery of pro-survival signals to injured brain tissue, increase in musculoskeletal coordination following stroke, improvements to consciousness following stroke, improvement to neurologic function following stroke, and improvement in motor function following stroke.

The method of treating ischemic stroke comprises administering a therapeutically effective amount of a chimeric protein by bolus injection to a subject in need thereof. The method of treating ischemic stroke comprises administering a pharmaceutical composition comprising a therapeutically effective amount of a chimeric protein by bolus injection to a subject in need thereof. The chimeric protein comprises, in order from N-terminus: (a) an activator domain comprising a variant of human insulin-like growth factor IGF-1, wherein the variant of IGF-1 comprises an amino acid sequence as set forth in SEQ ID NO: 2, (b) a half-life modulator comprising a variant of human serum albumin (HSA), wherein the variant of HSA comprises an amino acid sequence as set forth in SEQ ID NO: 15, and (c) a targeting domain comprising a variant of human Annexin 5 (AnxV), wherein the variant of AnxV comprises an amino acid sequence as set forth in SEQ ID NO: 10, optionally with a substitution to serine at position 315. Administration of the chimeric protein results in at least one of the following: mitigation of oxidative damage in primary cortical cultures, repair or acceleration of repair of blood brain barrier, reduction of oedema, reduction of infarct volume, reduction of blood brain barrier permeability, localization to injured brain tissue, increase in motor function following stroke.

In some embodiments, the variant of IGF-1 decreases activation of the IGF-1 receptor relative to the wild-type IGF-1. The variant of IGF-1 comprises E3R and Y31A substitutions relative to wild type human IGF-1.

The variant of human Annexin 5 comprises the amino acids 2-320 corresponding to wild type human Annexin 5 and comprises R63A, K70A, K101A, E138A, D139G, N160A, C316A substitutions relative to wild type human Annexin 5.

The variant of human serum albumin comprises the amino acids 26-609 corresponding to wild type human serum albumin and comprises C58S and N527Q substitutions relative to wild type human serum albumin.

In some embodiments, the chimeric protein is IGF1(E3R/Y31A)_lk7_HSA26-609(C58S/N527Q)_lk7_AnxV2-320(R63A/K70A/K101A/E138A/D139G/N160A/C316A). In some embodiments, the linker lk7 comprises - Gly-Ser-Gly-Gly-Gly-Ser-Gly.

In some embodiments, the chimeric protein is selectively targeted to cells comprising a target molecule phosphatidylserine and exhibits activation of the IGF-1 receptor at least twice as strong on cells containing the target molecule compared to cells that do not contain the target molecule as measured by phosphorylation of serine/threonine protein kinase B (AKT).

In some embodiments, administration is at the first presentation of a neurovascular disease.

In some embodiments, 0.01 mg/kg to 100 mg/kg per dose is administered to the subject in need thereof. In some embodiments, up to 200 mg/kg of the chimeric protein is administered to the subject in need thereof.

### Kits

Described herein are unit doses and kits. In some embodiments, the composition is provided in a kit. The kit may comprise a supply of individual unit doses comprising the chimeric protein; and instructions for use. In some embodiments, each unit dose is supplied in a suitable container or prefilled package, such as syringes, ampoules or vials. In some embodiments, each kit has a sufficient number of containers or unit dose to treat an individual patient for the suitable numbers of days for a course of treatment.

In some embodiments, the ingredients of the kit are supplied either separately or mixed together in unit dose, for example, as a dry lyophilized powder or water free concentrate in a hermetically sealed container such as an ampoule, vial, syringe or sachette indicating the quantity of active agent. Where the active agent(s) are lyophilized, an ampoule of sterile water or saline for injection can be provided so that the active agent(s) may be mixed prior to injection.

In some embodiments, the kit comprises a plurality of suitable containers, such as vials, and instructions for use. The vials may be fitted with a rubber septum to allow syringe entry for withdrawal of the vial contents. In some embodiments, each vial comprises between 1 and 50 ml extractable volume of the liquid formulation or any volume therebetween. In some embodiments, each vial comprises between 1 and 10 ml extractable volume of the liquid formulation. In some embodiments, each vial comprises 5 ml extractable volume of the liquid formulation. In some embodiments, the kit comprises a plurality of vials with different volume of the liquid formulation, wherein one or more of the plurality of vials comprises a different amount of the chimeric protein. In some embodiments, the plurality of containers comprises a liquid formulation comprising the chimeric protein to be administered in a volume ranging from 1 ml to 50 ml, or any volume therebetween. In some embodiments, the solution comprises about 20 mg/ml of chimeric protein. In some embodiments, the plurality of containers comprises a liquid formulation comprising from 20 mg to about 1g of the chimeric protein to be administered in a volume ranging from 1 ml to 50 ml or any volume therebetween. In some embodiments, the liquid formulation is ready for injection. In some embodiments, the liquid formulation is diluted into standard IV fluids (e.g., normal saline, 5% dextrose in normal saline, or 10% dextrose in water).

In some embodiments, the volume of the vial in the kit is between 1 mL to 50 mL, corresponding to 20 mg to 1 g of chimeric protein, such as scp776.

The kits described herein may further include other materials desirable from a commercial and user standpoint, including other buffers, diluents, filters, needles, syringes, and package inserts with instructions for performing any methods described herein.

In some embodiments, the instructions for use states to intravenously or intraarterially administer a unit dose from the supply once a day from the supply. In some embodiments, the instructions for use states to intravenously or intraarterially administer a unit dose from the supply two, three or more times a day from the supply. In some embodiments, the instructions for use states to intrathecally administer a unit dose from the supply once a day from the supply. In some embodiments, the instructions for use states to intrathecally administer a unit dose from the supply two, three or more times a day from the supply.

In some embodiments, the supply supports a single course of treatment. In some embodiments, the supply comprises 2, 3, 4, 5, 6, 7, 8, 9, 10, 11, 12, 13, 14 or more individual unit dose (e.g. vials). In some embodiments, the supply comprises 8 individual unit dose (e.g. vials)for a four day treatment course to account for variation in weight of patients and for convenience. In some embodiments, the supply comprises 14 individual unit dose (e.g. vials) for a seven days treatment course to account for variation in weight of patients and for convenience.

In some embodiments, the individual unit dose can be contained in one or more package and are optionally labelled "Dose 1", "Dose 2", "Dose 3" etc... or "Day 1", "Day 2", Day 3" etc...

In some embodiments, the unit dose that is to be administered on day 2 (referred herein as second dose comprises from about 85% to about 95% (e.g. about 90%) of the amount of the chimeric protein present in the unit dose that is to be administered on day 1 (referred herein as first dose), the unit dose that is to be administered on day 3 (referred herein as third dose) comprises from about 65% to about 85% (e.g. about 70%) of the amount of the chimeric protein present in the second dose and an amount that is lower than the second dose, the unit dose that is to be administered on day 4 comprises from about 45% to about 65% (e.g. about 50%) of the amount of the chimeric protein present in the first dose and an amount that lower than the third dose, the unit dose that is to be administered on day 5 comprises from about 35% to about 45% (e.g. about 40%) of the amount of the chimeric protein present in the first dose and an amount that lower than the fourth dose, the unit dose that is to be administered on day 6 comprises from about 25% to about 35% (e.g. about 30%) of the amount of the chimeric protein present in the first dose and an amount that lower than the fifth dose, and the unit dose that is to be administered on day 7 comprises from about 15% to about 25% (e.g. about 20%) of the amount of the chimeric protein present in the first dose and an amount that lower than the sixth dose

In some embodiments, the kit comprises a total dose of about 5 to 100 mg/kg of the chimeric proteins described herein for administration over a period of 4 days, 5 days, 6 days, or 7 days. In some embodiments, the kit comprises a total dose of about 5 to 100 mg/kg of the chimeric proteins described herein for administration over a period of 7 days. In some embodiments, the kit comprises a total dose of about 5 to 90 mg/kg of the chimeric proteins described herein for administration over a period of 4 days, 5 days, 6 days, or 7 days. In some embodiments, the kit comprises a total dose of about 5 to 90 mg/kg of the chimeric proteins described herein for administration over a period of 7 days. In some embodiments, the kit comprises a total dose of about 5 to 80 mg/kg of the chimeric proteins described herein for administration over a period of 4 days, 5 days, 6 days, or 7 days. In some embodiments, the kit comprises a total dose of about 5 to 80 mg/kg of the chimeric proteins described herein for administration over a period of 7 days. In some embodiments, the kit comprises a total dose of about 5 to 70 mg/kg of the chimeric proteins described herein for administration over a period of 4 days, 5 days, 6 days, or 7 days. In some embodiments, the kit comprises a total dose of about 5 to 70 mg/kg of the chimeric proteins described herein for administration over a period of 7 days. In some embodiments, the kit comprises a total dose of about 5 to 60 mg/kg of the chimeric proteins described herein for administration over a period of 4 days, 5 days, 6 days, or 7 days. In some embodiments, the kit comprises a total dose of about 5 to 60 mg/kg of the chimeric proteins described herein for administration over a period of 7 days. In some embodiments, the kit comprises a total dose of about 5 to 50 mg/kg of the chimeric proteins described herein for administration over a period of 4 days, 5 days, 6 days, or 7 days. In some embodiments, the kit comprises a total dose of about 5 to 50 mg/kg of the chimeric proteins described herein for administration over a period of 7 days. In some embodiments, the kit comprises a total dose of about 5 to 40 mg/kg of the chimeric proteins described herein for administration over a period of 4 days, 5 days, 6 days, or 7 days. In some embodiments, the kit comprises a total dose of about 5 to 40 mg/kg of the chimeric proteins described herein for administration over a period of 7 days. In some embodiments, the kit comprises a total dose of about 5 to 30 mg/kg of the chimeric proteins described herein for administration over a period of 4 days, 5 days, 6 days, or 7 days. In some embodiments, the kit comprises a total dose of about 5 to 30 mg/kg of the chimeric proteins described herein for administration over a period of 7 days. In some embodiments, the kit comprises a total dose of about 5 to 20 mg/kg of the chimeric proteins described herein for administration over a period of 4 days, 5 days, 6 days, or 7 days. In some embodiments, the kit comprises a total dose of about 5 to 20 mg/kg of the chimeric proteins described herein for administration over a period of 7 days. In some embodiments, the kit comprises a total dose of about 5 to 10 mg/kg of the chimeric proteins described herein for administration over a period of 4 days, 5 days, 6 days, or 7 days. In some embodiments, the kit comprises a total dose of about 5 to 10 mg/kg of the chimeric proteins described herein for administration over a period of 7 days. In some embodiments, the kit comprises a total dose of about 5 to 10 mg/kg of the chimeric proteins described herein for administration over a period of 4 days. In some embodiments, the kit comprises a total dose of about 10 mg/kg of the chimeric proteins described herein for administration over a period of 4 days, 5 days, 6 days, or 7 days. In some embodiments, the kit comprises a total dose of about 10 mg/kg of the chimeric proteins described herein for administration over a period of 4 days. In some embodiments, the kit comprises a total dose of about 10 mg/kg of the chimeric proteins described herein for administration over a period of 7 days. In some embodiments, the kit comprises a total dose of about 5 mg/kg of the chimeric proteins described herein for administration over a period of 4 days, 5 days, 6 days, or 7 days. In some embodiments, the kit comprises a total dose of about 5 mg/kg of the chimeric proteins described herein for administration over a period of 7 days. In some embodiments, the kit comprises a total dose of about 5 mg/kg of the chimeric proteins described herein for administration over a period of 4 days.

In some embodiments, the kit comprises a total dose of about 100 to 500 mg/kg of the chimeric proteins described herein for administration over a period of 4 days, 5 days, 6 days, or 7 days. In some embodiments, the kit comprises a total dose of about 100 to 500 mg/kg of the chimeric proteins described herein for administration over a period of 7 days. In some embodiments, the kit comprises a total dose of about 100 to 400 mg/kg of the chimeric proteins described herein for administration over a period of 4 days, 5 days, 6 days, or 7 days. In some embodiments, the kit comprises a total dose of about 100 to 400 mg/kg of the chimeric proteins described herein for administration over a period of 7 days. In some embodiments, the kit comprises a total dose of about 100 to 300 mg/kg of the chimeric proteins described herein for administration over a period of 4 days, 5 days, 6 days, or 7 days. In some embodiments, the kit comprises a total dose of about 100 to 300 mg/kg of the chimeric proteins described herein for administration over a period of 7 days. In some embodiments, the kit comprises a total dose of about 100 to 200 mg/kg of the chimeric proteins described herein for administration over a period of 4 days, 5 days, 6 days, or 7 days. In some embodiments the kit comprises a total dose of about 100 to 200 mg/kg of the chimeric proteins described herein for administration over a period of 7 days. In some embodiments, the kit comprises a total dose of about 100 to 150 mg/kg of the chimeric proteins described herein for administration over a period of 4 days, 5 days, 6 days, or 7 days. In some embodiments, the kit comprises a total dose of about 100 to 150 mg/kg of the chimeric proteins described herein for administration over a period of 7 days. In some embodiments, the kit comprises a total dose of about 140 mg/kg of the chimeric proteins described herein for administration over a period of 4 days, 5 days, 6 days, or 7 days. In some embodiments, the kit comprises a total dose of about 140 mg/kg of the chimeric proteins described herein for administration over a period of 7 days.

In some embodiments, the instructions for use comprises dosing schedule treatment in a readable medium such as pamphlet or written information. In some embodiments, the readable medium comprises specifications of the active agents, preparation instructions, dosage variations, storage instructions, administration instructions, side effects of the pharmaceutical ingredients, contraindications for administration, stability data or any other information relevant to the use of the composition described herein for patient care or physician information.

Aspects of the disclosure relate to the use of pharmaceutical composition for the treatment of ischemic stroke, the pharmaceutical composition comprising a therapeutically effective amount of a chimeric protein described herein and a pharmaceutically acceptable carrier, wherein the pharmaceutical composition is administered in the form of bolus injection. The chimeric protein comprises, in order from N-terminus: (a) an activator domain comprising a variant of human insulin-like growth factor IGF-1, wherein the variant of IGF-1 comprises an amino acid sequence as set forth in SEQ ID NO: 2, (b) a half-life modulator comprising a variant of human serum albumin (HSA), wherein the variant of HSA comprises an amino acid sequence as set forth in SEQ ID NO: 15, and (c) a targeting domain comprising a variant of human Annexin 5 (AnxV), wherein the variant of AnxV comprises an amino acid sequence as set forth in SEQ ID NO: 10, optionally with a substitution to serine at position 315.

### EXAMPLES

The following Examples are offered by way of illustration and not by way of limitation. Unless otherwise specified, all reagents and solvents are of standard commercial grade and are used without further purification.

### Example 1. Scp776-mediated In Vitro Protection of Primary Cortical Cells from Oxidative Stress

### Methods:

### Preparation of Rat Mixed Cortical Cultures

Rat mixed cortical cultures were prepared from E18 Wistar rat embryos under sterile conditions. In summary, following anesthesia, the embryos were removed from the womb and decapitated. The cortices were dissected out and placed in a dish containing cold 10 mM HEPES and 10 µg/mL gentamicin (HBSS-A) buffer. The tissue was cut into small pieces, transferred to a 15 mL conical tube and digested with DNase and papain for 15 min at 37 oC. The cells were collected by centrifugation (1500 rpm, 5 min RT). The supernatant was removed and 1 mL of MEM (2 g/L glucose) supplemented with 2 mM glutamine, 10 µg/mL gentamicin, 10 % heat-inactivated fetal bovine serum (FBS-HI) and 10 % heat-inactivated horse serum (HS-HI) was added to the tube and the tissue was triturated with a pipette. The cells were counted and then plated on poly-L-lysine coated 48-well plates, at a cell density of 400,000 cells/well in the same medium. The plates were placed in a humidified 37 °C/5 % CO2, 95 % air incubator. After 3-4 h, the medium was changed to MEM (2 g/L glucose) supplemented with 2 mM glutamine, 10 µg/mL gentamicin and 5 % HS-HI (MEM+). After three days in vitro, the medium was replaced with fresh medium containing MEM (2 g/L glucose) supplemented with 2 mM glutamine, 10 µg/mL gentamicin and 5 % FBS-HI and 5 % HS-HI. On day 6 in vitro, unwanted cell division was inhibited by adding cytosine arabinoside (10 µM final concentration in MEM+) for 24 h. The next day, (day 7 in vitro), the medium was replaced with fresh MEM+.

### H2O2 Exposure

Culture wells that had a healthy cell monolayer were selected for experimental treatment on day 10 after isolation in vitro. The cell culture media was completely removed from the wells and scp776, positive control, or vehicle control (DPBS) were added into the wells in a volume of 270 µl in MEM+. After the pre-incubation period of 30 mins at 37 °C, 30 µl of 500 µM H2O2 (final 50 µM in wells) was added and cells were incubated for 60 min at 37 °C/ 5 % CO2, 95 % air) incubator. Afterwards, the H2O2 medium was removed and normal culture media (MEM+) containing the compounds or vehicle control (DBPS) were pipetted to the wells, and the plates were placed in a normoxic (37 °C/ 5 % CO2, 95 % air) incubator for an additional 23 h.

### Assessment of Cell Death by LDH Measurement

Cell death was assessed by quantifying the levels of lactate dehydrogenase (LDH) released in the culture media following 23 h of H2O2 exposure. After 20 h the culture media from all wells were collected and possible cell debris was removed by centrifugation (13,000 rpm, 3 min, 4 °C). A 100 µL aliquot from each sample was pipetted into a 96 well micro titer plate as duplicates, and an equal volume of LDH substrate was pipetted into the wells. The absorbance at 340 nm was measured immediately using a 3 min kinetic measurement protocol in a Multiskan MS ELISA reader. The change in absorbance/min was determined, which is directly proportional to the released LDH.

### Assessment of Neuronal Cell Counts by NeuN Immunocytochemistry

Neuronal cell counts were quantified by immunostaining the neurons with the neuronal specific antibody targeting NeuN. After removing the culture medium, the cells were fixed with 4 % formaldehyde in PBS for 30 min at RT then washed twice with PBS. The fixed cells were permeabilized and non-specific binding was blocked by 30 min incubation with blocking buffer (1 % bovine serum albumin and 0.3 % Triton-X-100 in PBS). The cells were incubated with the primary antibody, mouse anti-NeuN (Millipore, catalog # MAB377, 1: 1,000 dilution), for 24 h at RT, followed by washing then a 2 h incubation with the secondary antibody, biotinylated goat anti-mouse (Vector Laboratories, catalog # BA-9200 1:200) at RT. The wells were washed twice with PBS and the cells were then incubated for 2 h with the avidin-HRP complex (Vector Laboratories, catalog # PK-6100 1:400) and the color was developed with nickel-enhanced DAB (Vector Laboratories, catalog # SK-4100). Each well was imaged at four random locations using a brightfield AxioVert A1 microscope (Carl Zeiss) using an LD A-Plan 20x objective (NA ∞/1.0 (PS)) (Carl Zeiss). The captured images were exported as tiff files using the ZEN software (Carl Zeiss). The tiff images were used for neuronal count analysis using ImageJ v1.48e (NIH). The images were manually thresholded to delimit the NeuN stained neurons that were counted according to their size by using the Counting Particles function available in the ImageJ software. The NeuN-positive neurons were counted by an experimenter blind to the treatments. Altogether, 4 fields for each well were counted.

### Results

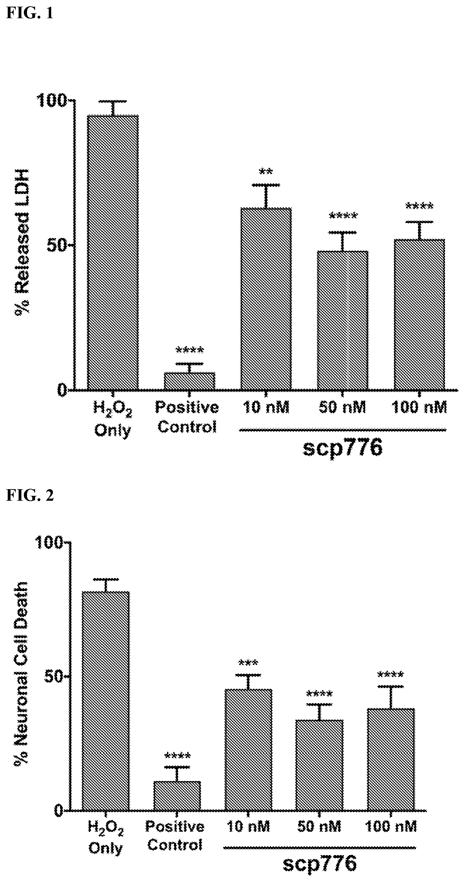

Primary mixed cortical cultures from E18 Wistar rat embryos were maintained under normal growth conditions for 10 days. The cells were then pre-treated with vehicle, positive control compound, or scp776 (10, 50 or 100 nM) for 30 minutes. Oxidative stress was then induced by addition of 50 µM H₂O₂. After 1 hour under H₂O₂-induced oxidative stress, the growth media was replaced with fresh media containing vehicle, positive control compound, or scp776 at different concentrations (10, 50 or 100 nM). After 23 hours, the media was removed and assayed for the presence of lactate dehydrogenase (LDH), an intracellular enzyme that is released into media upon cell death. FIG. 1 shows that a significant reduction in released LDH was observed in cultures treated with scp776 at 10, 50 or 100 nM compared to vehicle (H₂O₂ Only) (1-way ANOVA with Dunnett's multiple comparisons test; p = 0.0023 (10 nM), p < 0.0001 (50, 100 nM)).

Primary mixed cortical cultures from E18 Wistar rat embryos were maintained under normal growth conditions for 10 days. The cells were then pre-treated with vehicle, positive control compound, or scp776 at different concentrations (10, 50 or 100 nM) for 30 minutes. Oxidative stress was then induced by addition of 50 µM H₂O₂. After 1 hour under H₂O₂-induced oxidative stress, the growth media was replaced with fresh media containing vehicle, positive control compound, or scp776 (10, 50 or 100 nM). After 23 hours, the cells were fixed and stained for NeuN, a specific marker of neuronal nuclei, using immunocytochemical techniques. NeuN-positive cells were counted by light microscopy. FIG. 2 shows that a significant increase in NeuN positive cells (a reduction in cell death) was observed in cells treated with scp776 at 10, 50 or 100 nM compared to vehicle (H2O2 Only) (1-way ANOVA with Dunnett's multiple comparisons test; p = 0.0007 (10 nM), p < 0.0001 (50, 100 nM)).

### Conclusion

Scp776 protected against oxidative neuronal and glial cell death in vitro. Treatment of rat primary mixed cortical cultures with scp776 before, during and after stimulation of oxidative stress with hydrogen peroxide reduced cell death as measured by LDH release into cell media and by intact neuronal nuclei counting.

### Example 2. Scp776 Efficacy in the Rodent Model of Acute Ischemic Stroke (AIS)

### Methods

The transient Middle Cerebral Artery Occlusion (tMCAO) model creates an ischemic injury to brain that is representative of acute ischemic stroke. The duration of ischemia is controlled by the placement of an occluding filament, and the moment of reperfusion is determined by the removal of the filament. This mechanical model is analogous to standard revascularization modalities (e.g. - IV tissue plasminogen activator, intra-arterial thrombolysis, mechanical thrombectomy).

Each rat was anesthetized in an induction chamber (2 - 5% Isoflurane, in 70% N2O and 30% O2; flow 300 mL/min) and placed on its back on a surgery platform on a heating pad. The heating pad was set to 37°C and a rectal probe was inserted into the animal's rectum with help of a lubricant. Anesthesia was maintained at 1.5 - 2.5%.

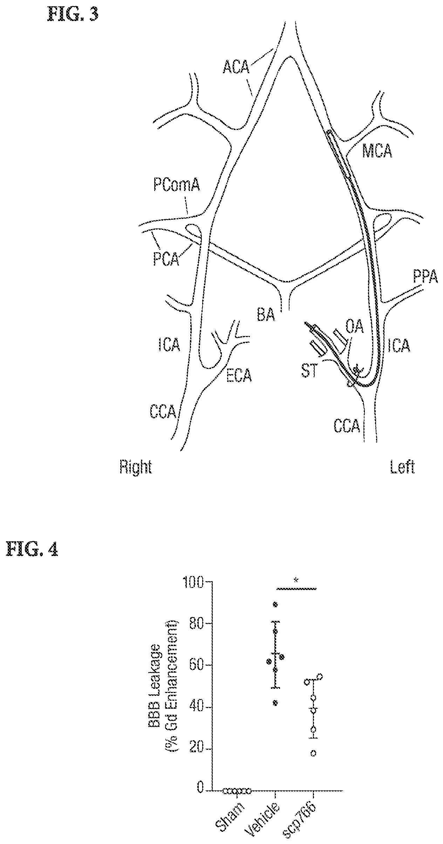

After midline skin incision, the right common carotid artery (CCA) was exposed, and the external carotid artery (ECA) was ligated distal from the carotid bifurcation. Filament with silicon-covered tip (Doccol filament 4-0, dia 0.185 mm, silicon 5 - 6 mm/dia 0.35 mm) was inserted 22 - 23 mm into the internal carotid artery (ICA) up to the origin of MCA (FIG. 3). After the operation, the rat was placed in a clean cage on a heating pad to wake up from the anesthesia. The filament was left in place for 90 min (120 minutes in some implementations).

For reperfusion, anesthesia was induced as described above, the animal was placed back on the heating pad and the rectal probe was inserted. The neck wound was carefully reopened, and the intraluminal filament was carefully removed to start reperfusion. After removal of the filament, the tissues were positioned back in place and the wound was sutured closed with the nylon thread. Pre-warmed 4 mL of 0.9% NaCl was given intraperitoneally (I.P.) to rehydrate the rat. After the operation, the rat was placed in a clean cage on a heating pad to wake up from the anesthesia. Finally, the rat was returned to its home cage and allowed free access to food and water.

Sham rats underwent identical procedures, including anesthesia regime, but without the filament insertion, ligation, and actual tMCAO occlusion.

Analytical methods used to assess scp776 efficacy in this model included: T2-weighted MRI (Infarct Size, Hemispheric Swelling), Gd-Enhanced MRI (Blood Brain Barrier (BBB) Integrity, Infarct Size), Tissue Homogenate ELISA (scp776 Accumulation), Automated Kinematic Gait Analysis.

### Dosage:

Rats were dosed with scp776 or vehicle in multiple dosing regimens. All dosing was intravenous (I.V.) bolus administration. In some implementations of the model, rats received a single I.V. dose of 25 mg/kg scp776 or vehicle 1-5 minutes prior to reperfusion. In other implementations, rats received 3 I.V. doses of 25 mg/kg scp776 or vehicle at the following times: 1-5 minutes prior to reperfusion, 4 hours post-reperfusion and 8 hours post-reperfusion.

### Results

IV administration of scp776 protects blood brain barrier integrity after ischemic stroke in rats. Sprague Dawley rats were subjected to the tMCAO model of ischemic stroke or sham procedure. At the time of reperfusion, tMCAO rats were dosed with an IV bolus of scp776 (25 mg/kg) or vehicle; sham rats were dosed with vehicle. Blood brain barrier (BBB) integrity was assessed by gadolinium-enhancement MRI 2 hours post-reperfusion. FIG. 4 shows the BBB leakage (percent Gd enhancement readout). Individual data points and means ± SD are shown for each group. Unpaired t-test showed a significant improvement (p = 0.014) in BBB integrity in the scp776 treated group, compared to vehicle.

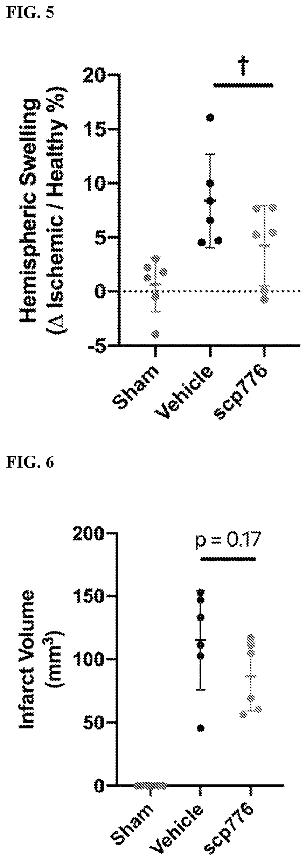

IV administration of scp776 reduces hemispheric swelling after ischemic stroke in rats. Sprague Dawley rats were subjected to the tMCAO model of ischemic stroke or sham procedure. At the time of reperfusion, tMCAO rats were dosed with an IV bolus of scp776 (25 mg/kg) or vehicle; sham rats were dosed with vehicle. Hemispheric swelling was assessed by T2-weighted MRI 2 hours post-reperfusion. FIG. 5 shows the hemispheric swelling (delta ischemic/healthy % readout). Individual data points and means ± SD are shown for each group. A trend of reduced hemispheric swelling was observed in animals that received scp776 compared to vehicle (Unpaired t-test; p = 0.10).

IV administration of scp776 reduces infarct size after ischemic stroke in rats (see FIG. 6). Sprague Dawley rats were subjected to the tMCAO model of ischemic stroke or sham procedure. At the time of reperfusion, tMCAO rats were dosed with an IV bolus of scp776 (25 mg/kg) or vehicle; sham rats were dosed with vehicle. Infarct size was assessed by T2-weighted MRI 2 hours post-reperfusion. Individual data points and means ± SD are shown for each group. A trend of reduced infarct size was observed in animals that received scp776 compared to vehicle (Unpaired t-test; p = 0.17).

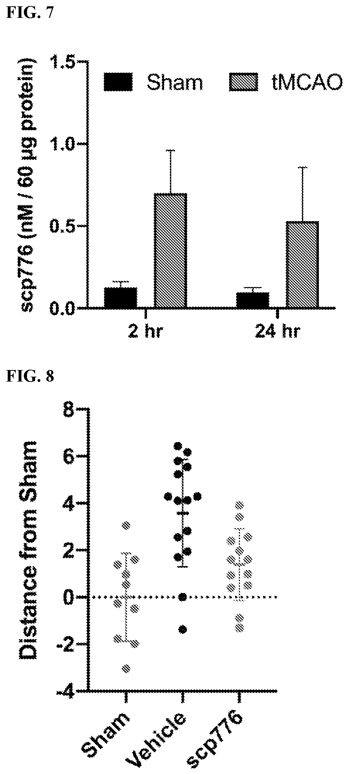

Scp776 treatment enhances recovery of function following ischemic stroke in rats (see FIG. 7). Sprague Dawley rats were subjected to the tMCAO model of ischemic stroke or sham procedure. At the time of reperfusion, tMCAO rats were dosed with an IV bolus of scp776 (25 mg/kg) or vehicle; sham rats were dosed with vehicle. A group of animals that were subjected to tMCAO received additional 25 mg/kg doses of scp776 at 4 and 8 hours post-reperfusion (Multiple Dose group). Fine motor kinematic analysis was performed on Day 10 post-tMCAO. Rats were analyzed in the MotoRater test using walking behavioral tasks (Step, Stride, Stance, Swing analyses, Limb Coordination). On the day of testing, the rats were marked in appropriate points of body, such as joints of limbs and parts of tail to ease the data analysis process. The movement data was captured using a high-speed camera (300 frames / second) from three different directions (from below and both sides). Different gait patterns and movements were analyzed using a custom-made automated analysis system. The analyzed parameters include: 1) general gait pattern parameters (stride time and speed, step width, stance and swing time during a stride, interlimb coordination), 2) body posture and balance (toe clearance, iliac crest and hip height, hind limb protraction and retraction, tail position and movement), and 3) fine motor skills (swing speed during a stride, jerk metric during swing phase, angle ranges and deviations of different joints, vertical and horizontal head movement). The analyzed parameters were combined into a single score per animal representing the deviation in overall movement from the average Sham (Healthy) animal; this parameter "Distance from Sham" is the y-axis of FIG. 8. The multiple dose group is labeled as scp776 in FIG. 8; the single dose group is not shown. FIG. 8 shows that the animals that received 3 doses of scp776 were significantly more similar to healthy animals than animals that received vehicle (1-way ANOVA with Tukey's Honest Significant Difference test; p = 0.0147).

### Conclusion

Scp776 showed efficacy in the rat tMCAO model of stroke by several direct measures of injury physiology. During the acute injury phase, 2 hours post-reperfusion and dosing, scp776-treated animals showed improved BBB integrity, reduced swelling of the infarcted hemisphere and reduced infarct volumes compared to vehicle treated animals. Coupled with the apparent beneficial activity in the injured brain, scp776 showed enhanced accumulation in injured brains at both 2 and 24 hours post-injury.

Scp776 also showed a functional benefit to tMCAO rats 10 days post-injury. Automated kinematic gait analysis defines a rat's walking stride in 97 parameters using high-speed video. A software package is used to cluster related parameters and evaluate the difference between those clusters in healthy animals and tMCAO animals. Animals that underwent the tMCAO procedure and received 3 doses of scp776 showed a significantly improved overall gait profile compared to animals that received vehicle.

Localization to injured brain tissue was unexpected. Whether due to the permeabilization of BBB in injured brain or active transport to regions of apoptosis, the capacity to deliver the chimeric protein such as scp776 to injured brain may open many therapeutic opportunities to treat central nervous brain disorders. In particular, the functional improvement in gait observed in scp776 treated animals 10 days following ischemic stroke was unexpected and surprising.

### Example 3: Efficacy study of scp776 in a Non-Human Primate Model (NHP) of Ischemic Stroke

### Methods

### 1. Description of the transient middle cerebral artery occlusion (tMCAO) Ischemic Stroke (IS) Model in Cynomolgus fascicularis (Macaca fascicularis)

Under anesthesia, the right eye globe was removed. Under an operating microscope (Kom300, Konan Medical, Hogo, Japan), the orbital content was dissected and excised. A window of approximately 10 mm in diameter was opened just anterior to the foramen of the optic nerve at the base of the skull. The main trunk of the right middle cerebral artery (MCA) was visible through the window, which is beneath dura matter. After opening the dura matter, tMCAO was performed using two microvascular clips (Mini #81, Sugita Aneurysm Clips, Mizuho Medical Corp., Tokyo, Japan), one on the proximal part of the main MCA trunk and the other on the distal-to-orbitofrontal branch. These clips were removed four (4) hours after MCA occlusion. After visual confirmation of re-establishment of MCA blood flow, the burr hole was closed using Clearfil New Bond (Kuraray Noritake Dental, Inc., Tokyo, Japan) and the incision was sutured. A pair of new clips was used for each macaque.

Any abnormal observations during clip placement and/or removal, such as vessel rupture or bleeding, was noted and reported.

### 2. Neurologic Deficit Scoring (NDS) Method

Twenty-four (24) hours (± 2 hrs.), 48 hrs. (± 2 hrs.), 72 hrs. (± 2 hrs.), 7, 10 and 14 days after occlusion, neurologic deficits were obtained according to the method described by Kito et al. (J. Neurosci. Meth., 2001; 105: 45-53).

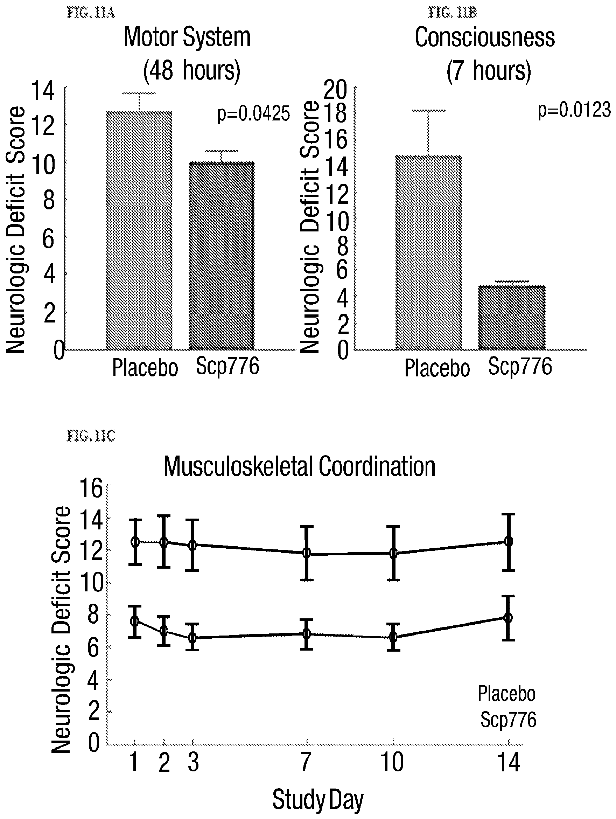

FIG. 11A shows the NDS motor system score at 48 hours (day 2) of placebo treated and scp776 treated animals.

FIG. 11B shows the NDS consciousness score at day 7 of placebo treated and scp776 treated animals.

FIG. 11C shows the NDS musculoskeletal coordination score at day 1, 2, 3, 7, 10, and 14 of placebo treated and scp776 treated animals.

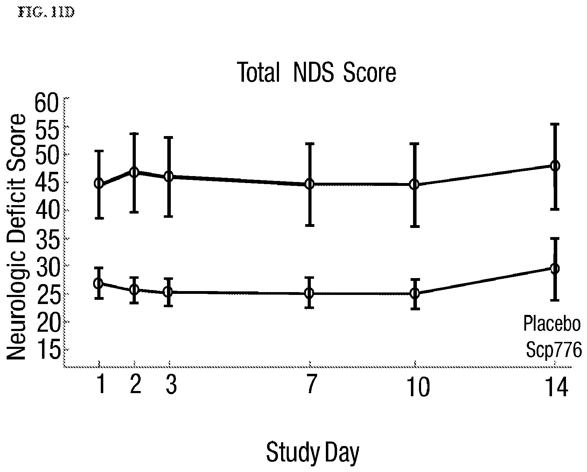

FIG. 11D shows the total NDS score at day 1, 2, 3, 7, 10, and 14 of placebo treated and scp776 treated animals.

### 3. Magnetic Resonance Imaging (MRI) Method

Brain images using MRI were obtained 4 hrs. after reperfusion (i.e. 8 hrs. after occlusion), 72 hrs. (±2 hrs.), and 14 days after occlusion.

During imaging, macaques were anesthetized with propofol (12-20 mg/kg; Maruishi PharmaceuticalCo., Osaka, Japan). Animals were fixed on a MRI bed (Signa Explorer 1.5T, GE Healthcare, Milwaukee, WI), and serial coronal images (6 mm thickness, vertical plane against orbitomeatal line) were be obtained. All imaging sequences consisted of the following:
- Diffusion-weighted imaging (DWI);
- Arterial spin labeling (ASL);
- Apparent diffusion coefficient (ADC);
- T1;
- Fluid-attenuated inversion-recovery (FLAIR); and
- T2-weighted imaging.

For the MRI conducted at the 8 hr. time point, in order to visualize blood brain barrier (BBB) disruption following treatment, contrast-enhanced T1-weighted imaging consisted of T1-weighted whole brain volume acquired before and about 5 min. after intravenous injection of gadobutrol contrast agent (1.0 mmol/mL, 0.2 mL/kg; Gadovist; Bayer HealthCare Pharmaceuticals, Osaka, Japan). All animals received a dose volume of 0.2 mL/kg.

Apparent diffusion coefficient (ADC) maps, cerebral blood flow (CBF), and perfusion deficit was generated with software (READY View, GE Healthcare) available on the MR scanner console for all MRIs done in the study.

The lesion volume (mm³) was calculated as the sum of the infarct area of each section and thickness (6 mm). Lesion volumes were calculated in cerebral cortex, white matter and basal ganglia and the total lesion volume was the sum of these volumes.

The area of BBB disruption (mm²) was delineated from each contrast-enhanced T1-weighted image using OsiriX MD version 12.0.0. BBB disruption was manually delineate using trace contrast-enhanced T1-weighted images. The volume (mm³) of BBB disruption was calculated as the sum of the areas of BBB disruption of each section (6 mm thickness).

### Dosage

All animals received one (1) dose of 1 ml/kg thirty minutes prior to release of the clips (or 3.5 hours after clip placement) and an additional four doses separated by 24 hours for a total of five (5) doses via Intravenous (i.v.) slow push over approximately 30 seconds. Placebo corresponds to vehicle alone (20 mM Tris, 7.5% w/v Sucrose, 0.02% w/v polysorbate 80, pH 7.5).

All animals received a total of five (5) doses. The dosage regimen is shown the table below. The dose 16 mg/kg corresponds to 16 mg/ml and the dose 4 mg/kg correspond to 4 mg/ml of the chimeric protein. The interval between administrations was no less than 20 hours and no more than 28 hours. Any instances where the interval between administrations falls outside of this range (20 - 28 hours) was reported.

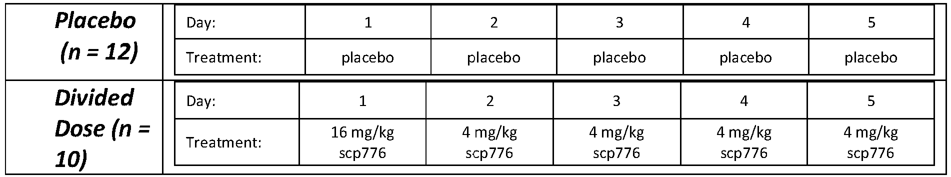

### Dextrose supplementation

In some embodiments, the animals being treated are continuously infused at a rate of 0.5 mL/kg/hr with dextrose to sustain euglycemia. Dextrose solution can comprise between about 5% to about 50% (w/v) dextrose in sterile water or saline solution.

### NDS, MRI, and Survival Results Summary

Animals were assessed using NDS scoring: The four systems for the NDS scoring system are Motor, Consciousness, Musculoskeletal coordination, and Sensory. The Total NDS score is the sum of the four systems.

Treatment with scp776 significantly improves neurologic function in three of the four systems that are assessed in the NDS scoring when compared with treatment with placebo.
- Significant improvements to Consciousness were observed at day 3 (not shown) and day 7 (see FIG. 11B).
- Significant improvements to Motor System were observed at day 2 (see FIG. 11A).
- Significant improvements to Musculoskeletal Coordination were observed at day 1, 2, 3, 7, 10, and 14 (See FIG. 11C).
- Improvement to total NDS score were observed at all time points. Significant improvements to total NDS score were observed at day 1 and day 3 (see FIG. 11D).

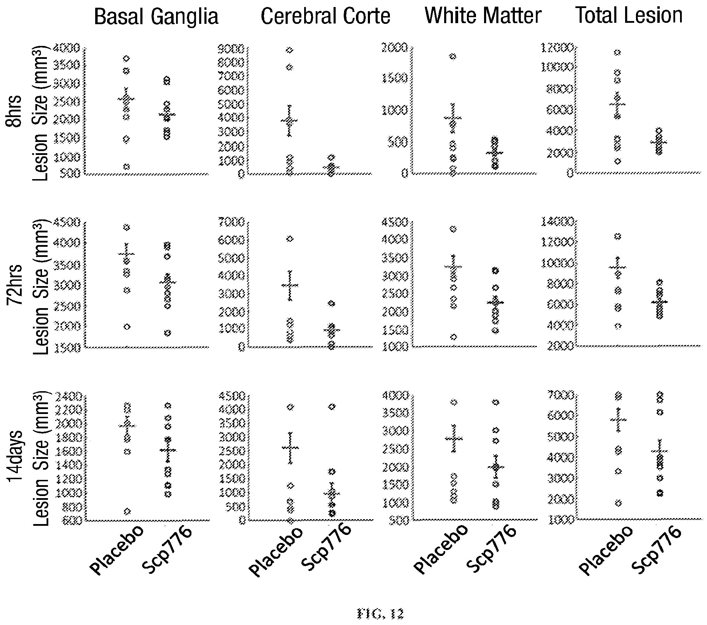

At all time points evaluated, treatment with scp776 resulted in a smaller lesion size or area than treatment with placebo (see FIG. 12).
- The reduction in lesion volume achieved statistical significance in the cerebral cortex and total lesion volumes at 8 hrs.
- Total lesion volume was significantly reduced at 72 hours by scp776 treatment.
- The volume of lesion in the cerebral cortex was significantly reduced by scp776 treatment at 14 days.

Scp776 treatment resulted in a statistically significant survival benefit. In the placebo group, 50% of the animals died as a result of the stroke injury, while only 10% of animals that received scp776 died prior to study completion. This survival difference, when assessed by the log rank method and chi-squared distribution, reveals a p-value of 0.039 (Bewick et al. Critical Care 2004, 8:389-394).

### Example 4 Treatment of Parkinson's Disease (PD), Amyotrophic lateral sclerosis (ALS), and Alzheimer's Disease (AD)

Parkinson's Disease (PD), Amyotrophic lateral sclerosis (ALS), and Alzheimer's Disease (AD) are characterized by progressive degeneration of the central or peripheral nervous systems. Each of these diseases is associated with impaired musculoskeletal coordination and motor system control. The model of Acute Ischemic Stroke in the non-human primates induces significant disfunction in both musculoskeletal coordination and motor control. The neuroprotectant activity of scp776 significantly reduces these disfunctions. The neuroprotectant activity of scp776 is independent of the type of insult that results in injured tissue. In chronic neurologic diseases, brain tissue is also injured and undergoes similar distress at the cellular level. Taken together, these results and the mode of disease progression, indicate that if scp776 were to be administered during symptomatic progression, the neuroprotective outcome observed in the model of acute ischemic stroke would translate to the chronic disease setting.

Experiments are conducted to examine the effect of scp776 on the treatment of Parkinson disease. Scp776 is dosed from about 0.01 mg/kg to about 20 mg/kg as often as daily. Administration results in improvements of, for example but not limited to, the measures of motor function and decrease in neurodegenerative symptoms, such as decreases of tremors.

Experiments are conducted to examine the effect of scp776 on the treatment of Amyotrophic lateral sclerosis (ALS). Scp776 is dosed from about 0.01 mg/kg to about 20 mg/kg as often as daily. Administration results in improvements of, for example but not limited to, measures of motor function and decreases on ALS symptoms such as autonomic dysfunction.

Experiments are conducted to examine the effect of scp776 on the treatment of Alzheimer's Disease (AD). Scp776 is dosed from about 0.01 mg/kg to about 20 mg/kg as often as daily. Administration results in improvements of, for example but not limited to, measures of cognitive function and decreases on AD symptoms such as memory loss and confusion.

## Claims

1. A pharmaceutical composition comprising a therapeutically effective amount of a chimeric protein and a pharmaceutically acceptable carrier for use in a method of treating ischemic stroke, the method comprising administering by bolus injection to a subject in need thereof the pharmaceutical composition,
wherein the chimeric protein comprises, in order from N-terminus: (a) an activator domain comprising a variant of human insulin-like growth factor IGF-1, wherein the variant of IGF-1 comprises an amino acid sequence as set forth in SEQ ID NO: 2, (b) a half-life modulator comprising a variant of human serum albumin (HSA), wherein the variant of HSA comprises an amino acid sequence as set forth in SEQ ID NO: 15, and (c) a targeting domain comprising a variant of human Annexin 5 (AnxV), wherein the variant of AnxV comprises an amino acid sequence as set forth in SEQ ID NO: 10, optionally with a substitution to serine at position 315,
wherein administration results in at least one of: mitigation of oxidative damage to cells of the cerebral cortex, repair or acceleration of repair of blood brain barrier, reduction of oedema, reduction of infarct volume, reduction of blood brain barrier permeability, targeted stimulation of the phosphorylation of serine/threonine protein kinase B (AKT) pathway by selective activation of the IGF-1 receptor in cells of injured brain tissue, targeted delivery of pro-survival signals to injured brain tissue, increase in musculoskeletal coordination following stroke, improvement to consciousness following stroke, improvement to neurologic function following stroke, and improvement in motor function following stroke.

2. The pharmaceutical composition for use according to claim 1, wherein the variant of IGF-1 decreases activation of the IGF-1 receptor relative to the wild type IGF-1.

3. The pharmaceutical composition for use according to claim 1, wherein the chimeric protein comprises a linker between the activator domain and the half-life modulator and a linker between the half-life modulator and the targeting domain, wherein the linker comprises -Gly-Ser-Gly-Gly-Gly-Ser-Gly.

4. The pharmaceutical composition for use according to any one of claims 1-3, wherein the chimeric protein is selectively targeted to cells comprising a target molecule phosphatidylserine and wherein the chimeric protein exhibits activation of the IGF-1 receptor at least twice as strong on cells containing the target molecule compared to cells that do not contain the target molecule as measured by phosphorylation of serine/threonine protein kinase B (AKT).

5. The pharmaceutical composition for use according to any one of claims 1-4, comprising administering within 72 hours of diagnosis of the ischemic stroke the pharmaceutical composition to the subject in need thereof.

6. The pharmaceutical composition for use according to any one of claims 1-4, comprising administering daily from about 0.01 mg/kg to about 20 mg/kg of the chimeric protein to the subject in need thereof.

7. The pharmaceutical composition for use according to any one of claims 1-4, comprising administering a total dose of from about 5 mg/kg to about 20 mg/kg of the chimeric protein to the subject in need thereof over a period of 4 to 7 days.

8. The pharmaceutical composition for use according to any one of claims 1-4, comprising administering descending doses of the chimeric protein to the subject in need thereof, optionally comprising administering to the subject in need thereof a first dose comprising from about 2 mg/kg to about 6 mg/kg of the chimeric protein on day 1, and a dose comprising from about 1 mg/kg to about 2 mg/kg one each of the following days.

9. The pharmaceutical composition for use according to any one of claims 1-8, comprising administering intravenously, intraarterially, or intrathecally the pharmaceutical composition.

10. The pharmaceutical composition for use according to any one of claims 1-4, wherein the composition is provided in a kit, the kit comprising a plurality of individual containers, each individual container comprising about 20 mg to about 1,000 mg of the chimeric protein.

11. The pharmaceutical composition for use according to any one of claims 1-10, wherein the chimeric protein comprises an amino acid sequence as set forth in SEQ ID NO: 24.

12. The pharmaceutical composition for use according to any one of claims 1-10, wherein the chimeric protein comprises an amino acid sequence as set forth in SEQ ID NO: 25.

## Patentansprüche

1. Pharmazeutische Zusammensetzung, umfassend eine therapeutisch wirksame Menge eines chimären Proteins und einen pharmazeutisch annehmbaren Träger, zur Verwendung in einem Verfahren zur Behandlung eines ischämischen Schlaganfalls, wobei das Verfahren das Verabreichen der pharmazeutischen Zusammensetzung durch Bolus-Injektion an ein Individuum mit Bedarf daran umfasst,
wobei das chimäre Protein in der Reihenfolge vom N-Terminus Folgendes umfasst: (a) eine Aktivatordomäne, umfassend eine Variante von menschlichem Insulin-ähnlichen Wachstumsfaktor IGF-1, wobei die Variante von IGF-1 eine Aminosäuresequenz wie in SEQ ID NO: 2 dargelegt umfasst, (b) einen Halbwertszeitmodulator, umfassend eine Variante von menschlichem Serumalbumin (HSA), wobei die Variante von HSA eine Aminosäuresequenz wie in SEQ ID NO: 15 dargelegt umfasst, und (c) eine abzielende Domäne, umfassend eine Variante von menschlichem Annexin 5 (AnxV), wobei die Variante von AnxV eine Aminosäuresequenz wie in SEQ ID NO: 10 dargelegt umfasst, wobei gegebenenfalls eine Substitution zu Serin an Position 315 vorhanden ist,
wobei die Verabreichung zu zumindest einem der Folgenden führt: der Linderung von oxidativer Schädigung von Zellen des zerebralen Cortex, der Reparatur oder Beschleunigung der Reparatur der Blut-Hirn-Schranke, der Reduktion von Ödemen, der Reduktion eines Infarktvolumens, der Reduktion der Durchlässigkeit der Blut-Hirn-Schranke, der abgezielten Stimulation der Phosphorylierung des Serin/Threonin-Proteinkinase-B- (AKT-) Wegs durch selektive Aktivierung des IGF-1-Rezeptors in Zellen von verletztem Hirngewebe, der abgezielten Zufuhr von Pro-Überlebenssignalen an das verletzte Hirngewebe, einer Erhöhung der muskuloskelettalen Koordination nach einem Schlaganfall, einer Verbesserung des Bewusstseins nach einem Schlaganfall, einer Verbesserung einer neurologischen Funktion nach einem Schlaganfall und einer Verbesserung der motorischen Funktion nach einem Schlaganfall.

2. Pharmazeutische Zusammensetzung zur Verwendung nach Anspruch 1, wobei die Variante von IGF-1 die Aktivierung des IGF-1-Rezeptors relativ zu dem Wildtyp-IGF-1 verringert.

3. Pharmazeutische Zusammensetzung zur Verwendung nach Anspruch 1, wobei das chimäre Protein einen Linker zwischen der Aktivatordomäne und dem Halbwertszeitmodulator und einen Linker zwischen dem Halbwertszeitmodulator und der abzielenden Domäne umfasst, wobei der Linker -Gly-Ser-Gly-Gly-Gly-Ser-Gly umfasst.

4. Pharmazeutische Zusammensetzung zur Verwendung nach einem der Ansprüche 1 bis 3, wobei das chimäre Protein selektiv auf Zellen abzielt, die ein Zielmolekül Phosphatidylserin umfassen, und wobei das chimäre Protein die Aktivierung des IGF-1-Rezeptors aufweist, die auf Zellen, die das Zielmolekül enthalten, verglichen mit Zellen, die das Zielmolekül nicht enthalten, zweimal so stark ist wie durch die Phosphorylierung der Serin/Threonin-Proteinkinase B (AKT) gemessen.

5. Pharmazeutische Zusammensetzung zur Verwendung nach einem der Ansprüche 1 bis 4, umfassend das Verabreichen der pharmazeutischen Zusammensetzung an das Individuum mit Bedarf daran innerhalb von 72 h nach der Diagnose des ischämischen Schlaganfalls.

6. Pharmazeutische Zusammensetzung zur Verwendung nach einem der Ansprüche 1 bis 4, umfassend das tägliche Verabreichen von etwa 0,01 mg/kg bis etwa 20 mg/kg des chimären Proteins an das Individuum mit Bedarf daran.

7. Pharmazeutische Zusammensetzung zur Verwendung nach einem der Ansprüche 1 bis 4, umfassend das Verabreichen einer Gesamtdosis von etwa 5 mg/kg bis etwa 20 mg/kg des chimären Proteins an das Individuum mit Bedarf daran über einen Zeitraum von 4 bis 7 Tagen.

8. Pharmazeutische Zusammensetzung zur Verwendung nach einem der Ansprüche 1 bis 4, umfassend das Verabreichen von abnehmenden Dosen des chimären Proteins an das Individuum mit Bedarf daran, gegebenenfalls umfassend das Verabreichen einer ersten Dosis, umfassend etwa 2 mg/kg bis etwa 6 mg/kg des chimären Proteins, an Tag 1 und einer Dosis, umfassend von etwa 1 mg/kg bis etwa 2 mg/kg, an jedem der darauffolgenden Tage.

9. Pharmazeutische Zusammensetzung zur Verwendung nach einem der Ansprüche 1 bis 8, umfassend das intravenöse, intraarterielle oder intrathekale Verabreichen der pharmazeutischen Zusammensetzung.

10. Pharmazeutische Zusammensetzung zur Verwendung nach einem der Ansprüche 1 bis 4, wobei die Zusammensetzung in einem Set bereitgestellt ist, wobei das Set eine Vielzahl von einzelnen Behältern umfasst, wobei jeder einzelne Behälter etwa 20 mg bis etwa 1.000 mg des chimären Proteins umfasst.

11. Pharmazeutische Zusammensetzung zur Verwendung nach einem der Ansprüche 1 bis 10, wobei das chimäre Protein eine Aminosäuresequenz wie in SEQ ID NO: 24 dargelegt umfasst.

12. Pharmazeutische Zusammensetzung zur Verwendung nach einem der Ansprüche 1 bis 10, wobei das chimäre Protein eine Aminosäuresequenz wie in SEQ ID NO: 25 dargelegt umfasst.

## Revendications

1. Composition pharmaceutique comprenant une quantité thérapeutiquement efficace d'une protéine chimérique et d'un support pharmaceutiquement acceptable pour utilisation dans un procédé de traitement d'un accident vasculaire cérébral ischémique, le procédé comprenant une administration par injection en bolus à un sujet qui en a besoin de la composition pharmaceutique,
dans laquelle la protéine chimérique comprend, dans l'ordre à partir de l'extrémité N-terminale : (a) un domaine activateur comprenant un variant du facteur de croissance de type insuline humain IGF-1, dans laquelle le variant d'IGF-1 comprend une séquence d'acides aminés telle que définie dans SEQ ID NO : 2, (b) un modulateur de demi-vie comprenant un variant de l'albumine sérique humaine (HSA), dans laquelle le variant de HSA comprend une séquence d'acides aminés telle que définie dans SEQ ID NO : 15, et (c) un domaine de ciblage comprenant un variant de l'annexine 5 humaine (AnxV), dans laquelle le variant d'AnxV comprend une séquence d'acides aminés telle que définie dans SEQ ID NO : 10, facultativement avec une substitution à la sérine en position 315,
dans laquelle l'administration entraîne au moins une parmi : une atténuation de dommage oxydatif aux cellules du cortex cérébral, une réparation ou une accélération de la réparation de la barrière hémato-encéphalique, une réduction d'œdème, une réduction de volume d'infarctus, une réduction de la perméabilité de la barrière hémato-encéphalique, une stimulation ciblée de la phosphorylation de la voie de la sérine/thréonine protéine kinase B (AKT) par activation sélective du récepteur d'IGF-1 dans des cellules du tissu cérébral blessé, une délivrance ciblée de signaux de pro-survie au tissu cérébral blessé, une augmentation de la coordination musculo-squelettique après un accident vasculaire cérébral, une amélioration de la conscience après un accident vasculaire cérébral, une amélioration de la fonction neurologique après un accident vasculaire cérébral et une amélioration de la fonction motrice après un accident vasculaire cérébral.

2. Composition pharmaceutique pour utilisation selon la revendication 1, dans laquelle le variant d'IGF-1 diminue l'activation du récepteur d'IGF-1 par rapport à l'IGF-1 de type sauvage.

3. Composition pharmaceutique pour utilisation selon la revendication 1, dans laquelle la protéine chimérique comprend un lieur entre le domaine activateur et le modulateur de demi-vie et un lieur entre le modulateur de demi-vie et le domaine de ciblage, dans laquelle le lieur comprend -Gly-Ser-Gly-Gly-Gly-Gly-Ser-Gly.

4. Composition pharmaceutique pour utilisation selon l'une quelconque des revendications 1 à 3, dans laquelle la protéine chimérique est ciblée sélectivement par rapport à des cellules comprenant une molécule cible de phosphatidylsérine et dans laquelle la protéine chimérique présente une activation du récepteur d'IGF-1 au moins deux fois plus forte sur des cellules contenant la molécule cible par comparaison à des cellules qui ne contiennent pas la molécule cible, telle que mesurée par phosphorylation de sérine/thréonine protéine kinase B (AKT).

5. Composition pharmaceutique pour utilisation selon l'une quelconque des revendications 1 à 4, comprenant une administration, dans les 72 heures suivant le diagnostic de l'accident vasculaire cérébral ischémique, de la composition pharmaceutique au sujet qui en a besoin.

6. Composition pharmaceutique pour utilisation selon l'une quelconque des revendications 1 à 4, comprenant une administration quotidienne d'environ 0,01 mg/kg à environ 20 mg/kg de la protéine chimérique au sujet qui en a besoin.

7. Composition pharmaceutique pour utilisation selon l'une quelconque des revendications 1 à 4, comprenant une administration d'une dose totale d'environ 5 mg/kg à environ 20 mg/kg de la protéine chimérique au sujet qui en a besoin sur une période de 4 à 7 jours.

8. Composition pharmaceutique pour utilisation selon l'une quelconque des revendications 1 à 4, comprenant une administration de doses décroissantes de la protéine chimérique au sujet qui en a besoin, comprenant facultativement une administration au sujet qui en a besoin d'une première dose comprenant d'environ 2 mg/kg à environ 6 mg/kg de la protéine chimérique le jour 1, et d'une dose comprenant d'environ 1 mg/kg à environ 2 mg/kg chacun des jours suivants.

9. Composition pharmaceutique pour utilisation selon l'une quelconque des revendications 1 à 8, comprenant une administration par voie intraveineuse, intra-artérielle ou intrathécale de la composition pharmaceutique.

10. Composition pharmaceutique pour utilisation selon l'une quelconque des revendications 1 à 4, dans laquelle la composition est fournie dans un kit, le kit comprenant une pluralité de récipients individuels, chaque récipient individuel comprenant environ 20 mg à environ 1 000 mg de la protéine chimérique.

11. Composition pharmaceutique pour utilisation selon l'une quelconque des revendications 1 à 10, dans laquelle la protéine chimérique comprend une séquence d'acides aminés telle que définie dans SEQ ID NO : 24.

12. Composition pharmaceutique pour utilisation selon l'une quelconque des revendications 1 à 10, dans laquelle la protéine chimérique comprend une séquence d'acides aminés telle que définie dans SEQ ID NO : 25.
